# EUROPEAN PATENT APPLICATION

(11) **EP 4 079 837 A1**
(43) Date of publication of application: **26.10.2022**
(21) Application number: 20870250.6
(22) Date of filing: 25.09.2020
(51) Int. Cl.: C12N 1/20, C12N 1/36, A23L 33/135, A61K 35/74, A61K 45/06, A61P 3/00, A61P 25/00, A61P 31/04, A61P 35/00, C12R 1/01

(54) **OBLIGATE ANAEROBIC HUMAN INTESTINAL MICROBE FOR CANCER TREATMENT, AND USE THEREOF**

(30) Priority: 25.09.2019 KR 20190118268
(71) Applicant: HealthBiome, Inc., Daejeon 34141 (KR); Kim, Byoung-Chan, Yuseong-gu, Daejeon 34141 (KR)
(72) Inventor: SONG, Jinhoi, Daejeon 34127 (KR); YOON, Jun Beom, Daejeon 34008 (KR); SON, Mi Jin, Daejeon 35225 (KR); SONG, Da Young, Daejeon 34119 (KR); KIM, Byoung-Chan, Daejeon 34141 (KR)
(74) Representative: V.O.
(86) International application number: PCT/KR2020/013159
(87) International publication number: WO 2021/060945

(57) **Abstract**

The present disclosure relates to a novel *Akkermansia* strain and a use thereof. The novel *Akkermansia* HB03 strain *(Akkermansia* sp. HB03) provided in the present disclosure lacks toxic genes and thus does not cause side effects in vivo, and also exhibits excellent anti-cancer activity. Therefore, it can be widely used in the development of agents for preventing or treating cancer.

<110>    HEALTHBIOME

<120>    Strict anaerobic human gut microbe for antitumor treatment and
         uses thereof

<130>    OPA20175-PCT

<150>    KR 10-2019-0118268
<151>    2019-09-25

<160>    2

<170>    KoPatentIn 3.0

<210>    1
<211>    287
<212>    PRT
<213>    Unknown

<220>
<223>    HB03-01


<400>    1
Ile Val Asn Ser Lys Arg Ser Glu Leu Asp Lys Lys Ile Ser Ile Ala
  1               5                  10                  15

Ala Lys Glu Ile Lys Ser Ala Asn Ala Ala Glu Ile Thr Pro Ser Arg
             20                  25                  30

Ser Ser Asn Glu Glu Leu Glu Lys Glu Leu Asn Arg Tyr Ala Lys Ala
         35                  40                  45

Val Gly Ser Leu Glu Thr Ala Tyr Lys Pro Phe Leu Ala Ser Ser Ala
     50                  55                  60

Leu Val Pro Thr Thr Pro Thr Ala Phe Gln Asn Glu Leu Lys Thr Phe
 65                  70                  75                  80

Arg Asp Ser Leu Ile Ser Ser Cys Lys Lys Lys Asn Ile Leu Ile Thr
                 85                  90                  95

Asp Thr Ser Ser Trp Leu Gly Phe Gln Val Tyr Ser Thr Gln Ala Pro
            100                 105                 110

Ser Val Gln Ala Ala Ser Thr Leu Gly Phe Glu Leu Lys Ala Ile Asn
        115                 120                 125

Ser Leu Val Asn Lys Leu Ala Glu Cys Gly Leu Ser Lys Phe Ile Lys
    130                 135                 140

Val Tyr Arg Pro Gln Leu Pro Ile Glu Thr Pro Ala Asn Asn Pro Glu
145                 150                 155                 160

Glu Ser Asp Glu Ala Asp Gln Ala Pro Trp Thr Pro Met Pro Leu Glu
                165                 170                 175

Ile Ala Phe Gln Gly Asp Arg Glu Ser Val Leu Lys Ala Met Asn Ala
            180                 185                 190

Ile Thr Gly Met Gln Asp Tyr Leu Phe Thr Val Asn Ser Ile Arg Ile
        195                 200                 205

Arg Asn Glu Arg Met Met Pro Pro Pro Ile Ala Asn Pro Ala Ala Ala
    210                 215                 220

Lys Pro Ala Ala Ala Gln Pro Ala Thr Gly Ala Ala Ser Leu Thr Pro
225                 230                 235                 240

Ala Asp Glu Ala Thr Ala Pro Ala Ala Pro Ala Ile Gln Gln Val Ile
                245                 250                 255

Lys Pro Tyr Met Gly Lys Glu Gln Val Phe Val Gln Val Ser Leu Asn
            260                 265                 270

Leu Val His Phe Asn Gln Pro Lys Ala Gln Glu Pro Ser Glu Asp
        275                 280                 285

<210>    2
<211>    127
<212>    PRT
<213>    Unknown

<220>
<223>    HB03-03

<400>    2
Ile Pro Glu Ser Ser Val Pro Ala Ala Ala Val Ser Ser Val Lys Gly
  1               5                  10                  15

Ala Tyr Pro Asn Ala Gly Pro Ile Glu Trp Asp Ser Glu Met His Gly
            20                  25                  30

Met Glu Arg Ile Tyr Glu Ala Glu Phe Arg Ile Asn Gly Phe Gln Phe
        35                  40                  45

Asp Val Lys Ile Ala Pro Asp Gly Lys Ile Val Arg Ile Lys Glu Glu
    50                  55                  60

Ile Ala Ala Ser Ser Leu Pro Glu Ala Val Arg Ala Ser Val Leu Lys
 65                  70                  75                  80

Arg Tyr Pro Gly Cys Arg Ile Lys Glu Ala Glu Lys Ile Thr Glu Gly
                85                  90                  95

Glu Leu Val Arg Tyr Lys Val Glu Val Ala Asp Ser Arg Asp Asp Tyr
            100                 105                 110

Asp Val Leu Ile Ala Pro Asp Gly Asn Ile Leu His Val Asp Arg
        115                 120                 125

## Description

### [Technical Field]

The present disclosure relates to a novel *Akkermansia* HB03 strain belonging to the genus *Akkermansia* of the family *Verrucomicrobiales,* which is an obligate anaerobic human intestinal microbe.

### [Background Art]

An *Akkermansia* sp. strain is a Gram-negative, obligate anaerobic, human intestinal mucin-degrading bacterium. The *Akkermansia* sp. strain is known to be non-motile, non-spore-forming (Yuji Naito, et al., A next-generation beneficial microbe: Akkermansia muciniphila, J Clin Biochem Nutr. 2018 Jul; 63(1): 33-35.) and oval-shaped. It can use mucin as a sole carbon and nitrogen source and can be cultured under a strict anaerobic condition in a medium containing intestinal mucin. In addition, it can proliferate (colonize) in the gastrointestinal tract of various animal species.

It is known that intestinal microbes play an important role in the regulation of immune response against various types of tumors and have the potential of suppressing tumor cells and enhancing immunity in the tumor ecosystem. Therefore, they are useful for treatment of tumors.

Cancer is one of the main causes of death in adults globally. Although therapeutic performance has improved with the development of surgical techniques and the introduction of anti-cancer radiation therapy, about half of the diagnosed patients still die of cancer. For this reason, cancer is considered one of the major issues in many countries. Accordingly, the development of a method for treating and preventing cancer is needed urgently.

### [Disclosure]

### [Technical Problem]

The inventors of the present disclosure have made consistent efforts to discover a novel strain belonging to the genus *Akkermansia.* As a result, they have found a novel strain in the genus *Akkermansia,* which is distinguished from the previously known *Akkermansia muciniphila* strain, and completed the present disclosure by identifying that the strain exhibits superior anti-cancer activity as compared to the *Akkermansia muciniphila* strain.

### [Technical Solution]

The present disclosure is directed to providing a novel *Akkermansia* HB03 strain *(Akkermansia* sp. HB03) deposited with the accession number KCCM12318P.

The present disclosure is also directed to providing a pharmaceutical composition for preventing or treating cancer, which contains one or more selected from a group consisting of the novel *Akkermansia* HB03 strain, an inactivated strain thereof, a culture thereof, a vesicle thereof, a proteome thereof and a metabolome thereof.

The present disclosure is also directed to providing a method for preventing or treating cancer, which includes a step of administering the pharmaceutical composition to an individual.

The present disclosure is also directed to providing a food composition for preventing or alleviating cancer, which contains one or more selected from a group consisting of the novel *Akkermansia* HB03 strain, an inactivated strain thereof, a culture thereof, a vesicle thereof, a proteome thereof and a metabolome thereof.

### [Advantageous Effects]

A novel *Akkermansia* HB03 strain provided in the present disclosure lacks toxic genes and thus does not cause side effects in vivo. And, a composition containing the strain or a vesicle, a metabolome or an inactivated strain derived from the strain exhibits superior effect of inhibiting tumor growth and exhibits remarkably superior effect of inhibiting tumor when co-administered with another anti-cancer agent, particularly an immune checkpoint inhibitor. In addition, a protein derived from the strain as a metabolome also exhibits the effect of effectively inhibiting cancer cell growth and inhibiting tumor formation and progression. Accordingly, the strain can be widely used in the development of agents for preventing or treating cancer.

### [Brief Description of Drawings]

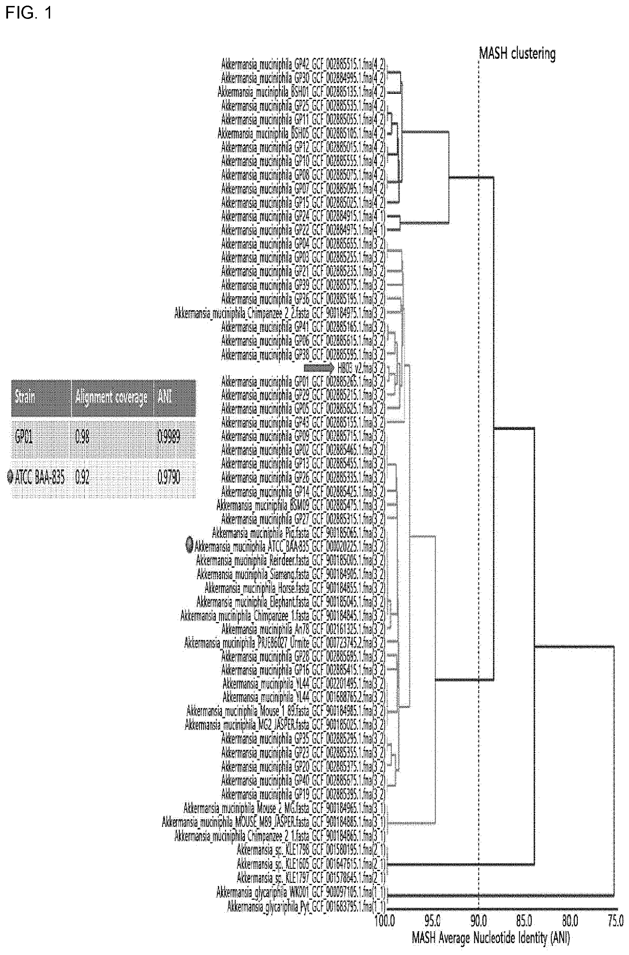

FIG. 1 shows a result of searching for the genome base sequences of a total of 66 *Akkermansia* species from NCBI and conducting clustering based on the average nucleotide identity of MASH genome base sequences using dRep.

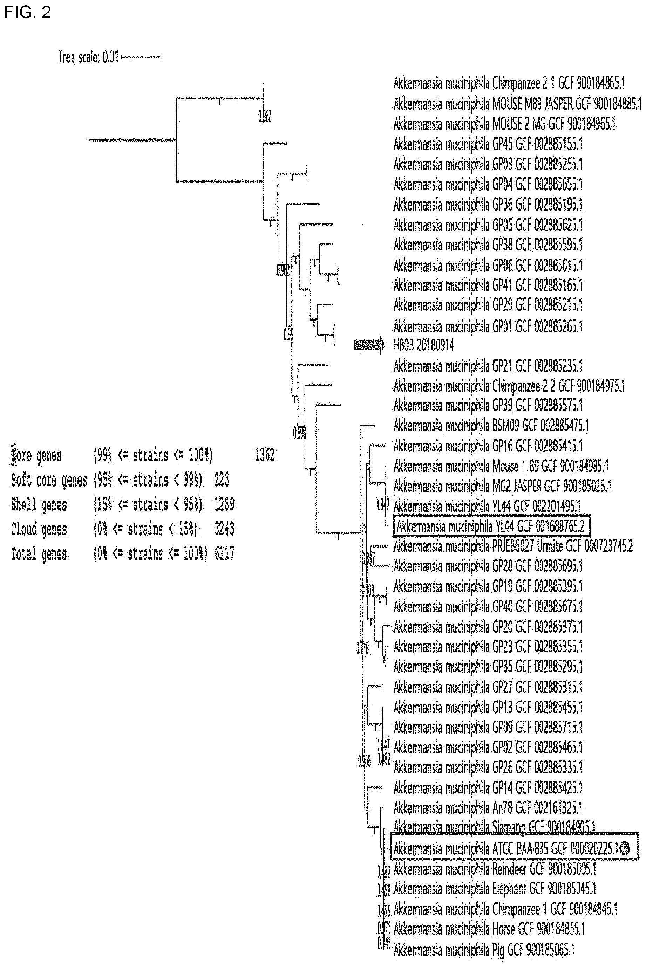

FIG. 2 shows a result of drawing the approximately maximum-likelihood tree for *Akkermansia* strains.

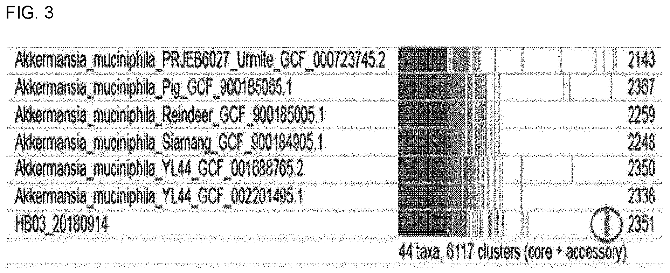

FIG. 3 shows a result of extracting 120 strain-specific genes from a gene presence/absence plot.

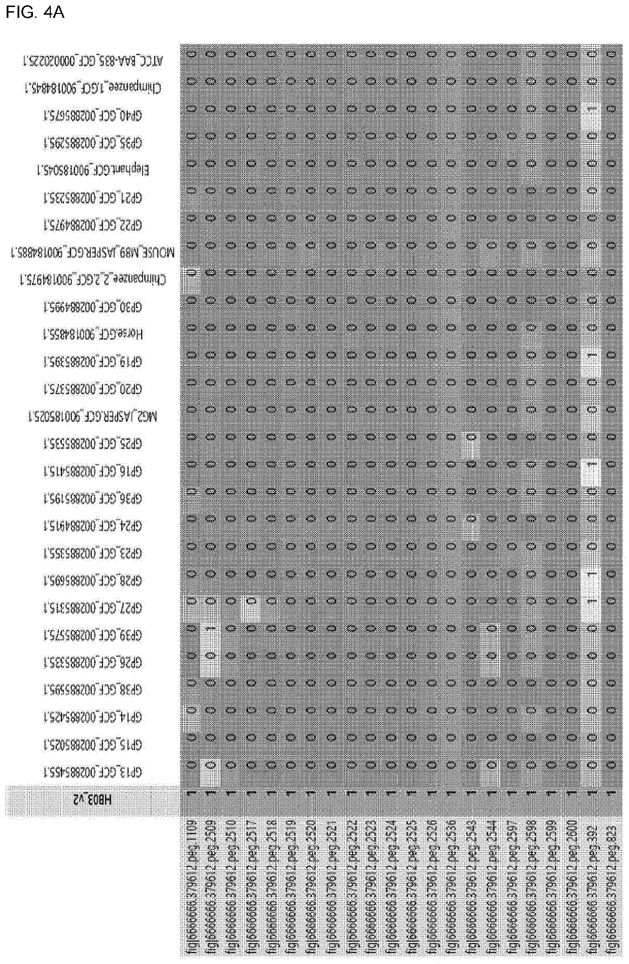

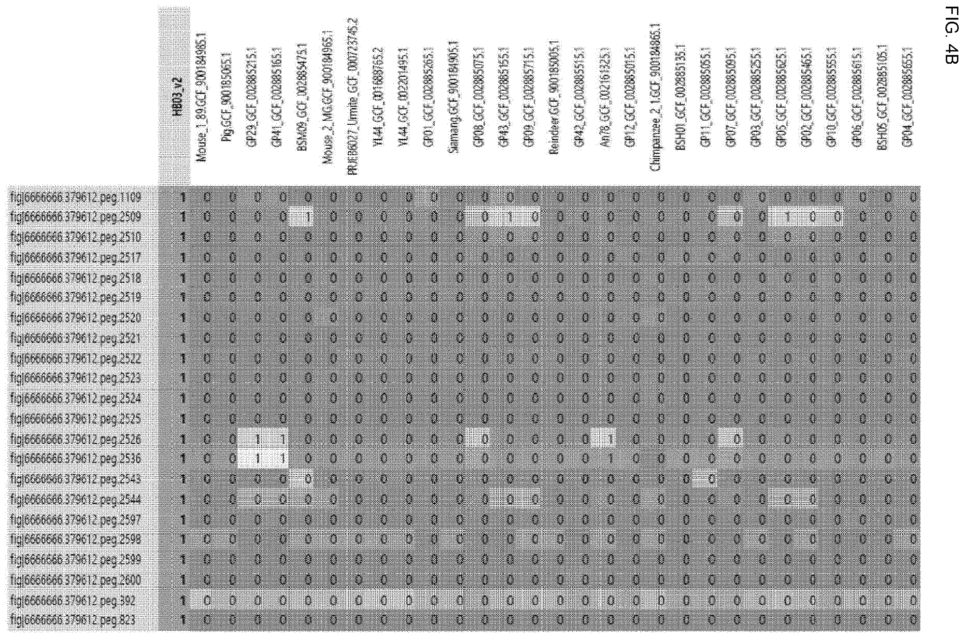

FIGS. 4a and 4b show a result of checking the presence of genes by calculating blast score ratios (BSRs) using LS-BSRs (large-scare blast score ratios).

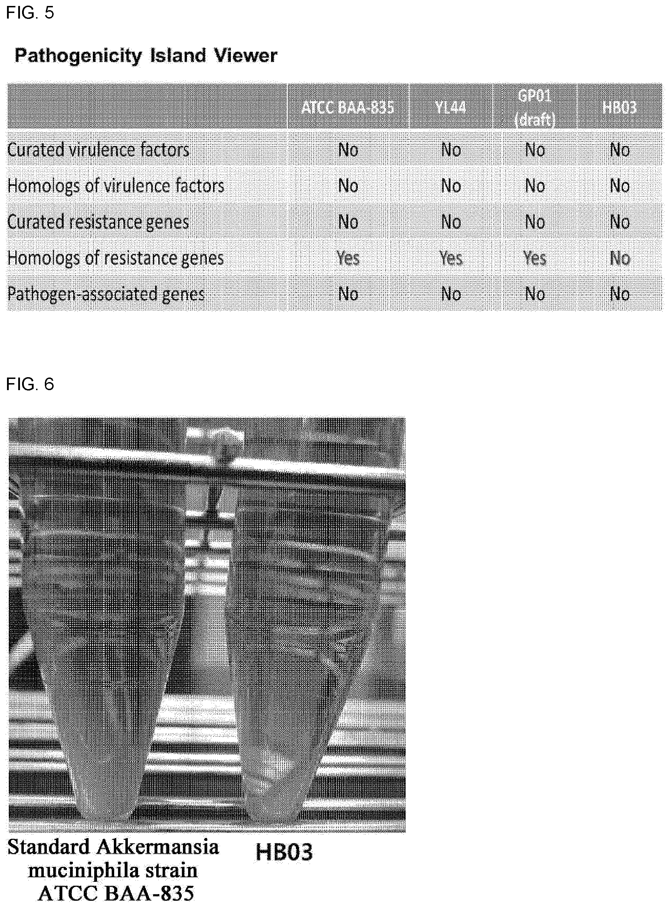

FIG. 5 shows a result of identifying the presence of pathogenicity islands (PAIs) in an HB03 strain.
FIG. 6 shows a result of investigating the degree of sedimentation of an HB03 strain and an *Akkermansia muciniphila* ATCC BAA-835 strain after centrifuging the strain culture at 7,000 rpm. Although the HB03 strain was completely separated from the supernatant, the *Akkermansia muciniphila* strain was not separated.

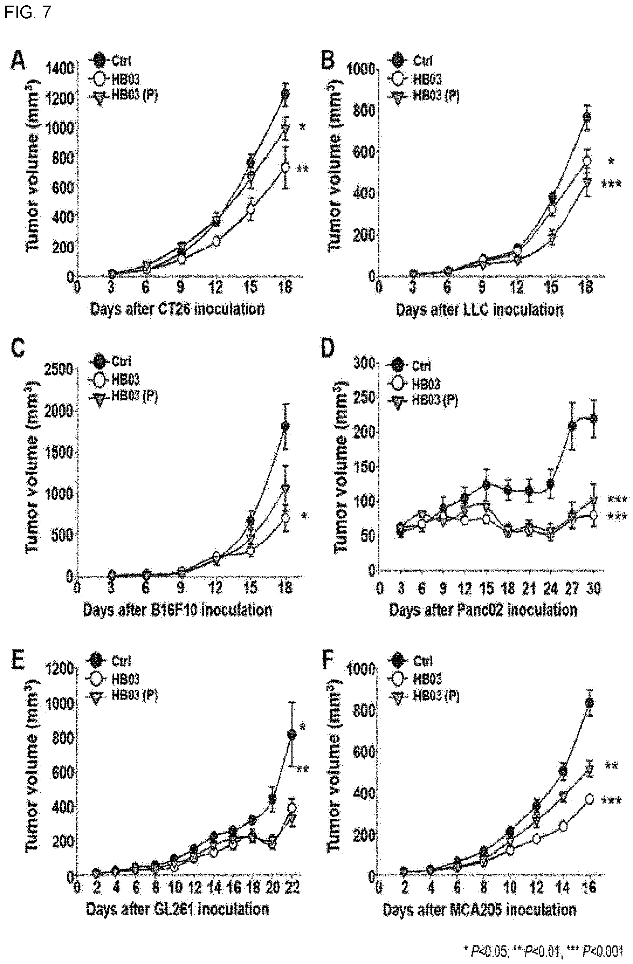

FIG. 7 shows a result of post-treating (administration after tumor inoculation) mice bearing tumors with HB03 and HB03 (P) and analyzing the difference in tumor growth with a control group. (A) Tumor growth kinetics was observed after inoculating BALB/c with CT26 colorectal cancer cells and then treating with HB03 and HB03 (P) every day from day 6 to day 18. (B) Tumor growth kinetics was observed after inoculating C57BL/6 JAX mice with LLC lung cancer cells and then treating with HB03 and HB03 (P) every day from day 6 to day 18. (C) Tumor growth kinetics was observed after inoculating C57BL/6 JAX mice with B16F10 melanoma cells and then treating with HB03 and HB03 (P) every day from day 6 to day 18. (D) Tumor growth kinetics was observed after inoculating C57BL/6 JAX mice with Panc02 pancreatic cancer cells and then treating with HB03 and HB03 (P) every day from day 6 to day 18. (E) Tumor growth kinetics was observed after inoculating C57BL/6 JAX mice with GL261 glioma cells and then treating with HB03 and HB03 (P) every day from day 6 to day 18. (F) Tumor growth kinetics was observed after inoculating C57BL/6 JAX mice with MCA205 sarcoma cells and then treating with HB03 and HB03 (P) every day from day 6 to day 18.

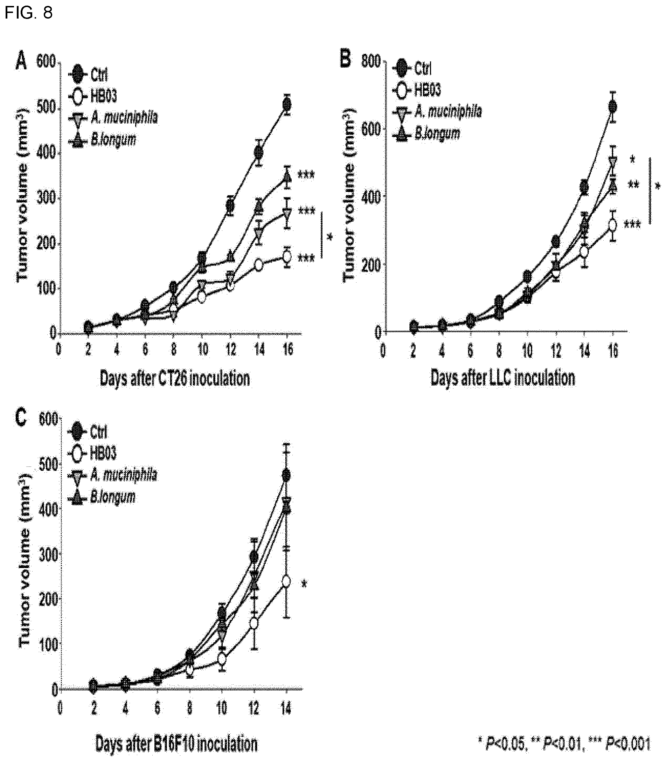

FIG. 8 shows a result of post-treating (administration after tumor inoculation) mice bearing tumors with HB03, *Akkermansia muciniphila* or *Bifidobacterium longum* and analyzing the difference in tumor growth with a control group. (A) Tumor growth kinetics was observed after inoculating BALB/c mice with CT26 colorectal cancer cells and then treating with HB03, *Akkermansia muciniphila* or *Bifidobacterium longum* from day 5 to day 16. (B) Tumor growth kinetics was observed after inoculating C57BL/6 JAX mice with LLC lung cancer cells and then treating with HB03, *Akkermansia muciniphila* or *Bifidobacterium longum* from day 5 to day 16. (C) Tumor growth kinetics was observed after inoculating C57BL/6 JAX mice with B16F10 melanoma cells and then treating with HB03, *Akkermansia muciniphila* or *Bifidobacterium longum* from day 5 to day 14.

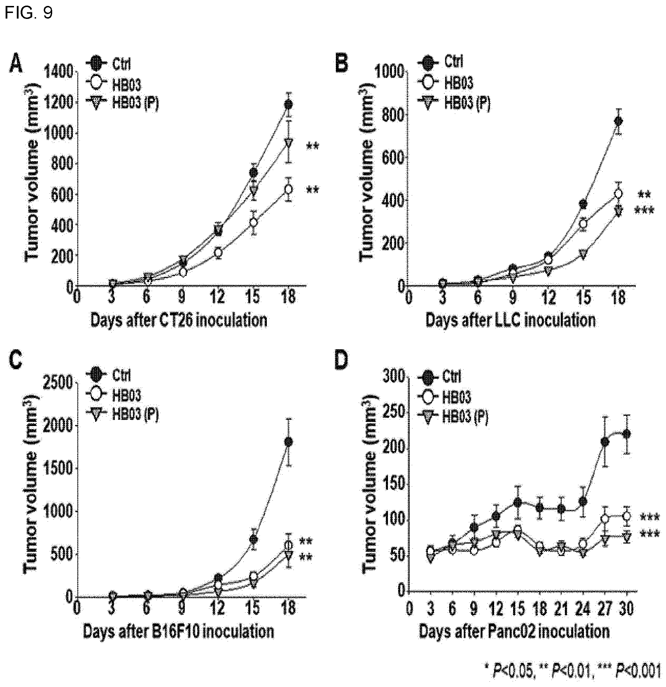

FIG. 9 shows a result of pre-treating (administration before tumor inoculation) mice bearing tumors with HB03 and HB03 (P) and analyzing the difference in tumor growth with a control group. (A) Tumor growth kinetics was observed after treating with HB03 and HB03 (P) every day from day 7 to day 18 before inoculating BALB/c mice with CT26 colorectal cancer cells. (B) Tumor growth kinetics was observed after treating with HB03 and HB03 (P) every day from day 7 to day 18 before inoculating C57BL/6 JAX mice with LLC lung cancer cells. (C) Tumor growth kinetics was observed after treating with HB03 and HB03 (P) every day from day 7 to day 18 before inoculating C57BL/6 JAX mice with B16F10 melanoma cells. (D) Tumor growth kinetics was observed after treating with HB03 and HB03 (P) every day from day 7 to day 18 before inoculating C57BL/6 JAX mice with Panc02 pancreatic cancer cells.

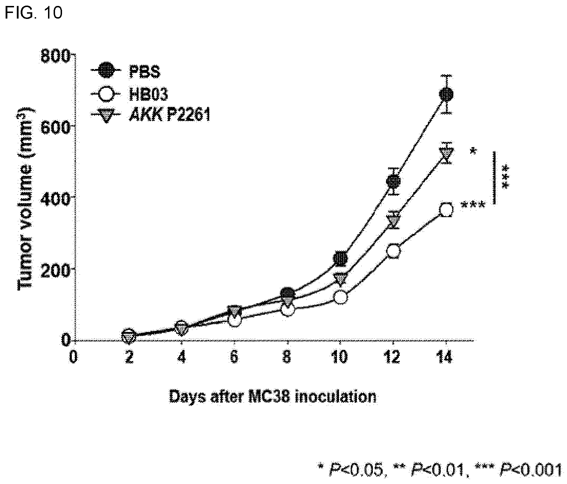

FIG. 10 shows a result of post-treating (administration after tumor inoculation) mice bearing tumors with HB03 or *Akkermansia muciniphila* (AKK P2261) and analyzing the difference in tumor growth with a control group. Tumor growth kinetics was observed after inoculating C57BL/6 JAX mice with MC38 colorectal cancer cells and then treating with HB03 or *Akkermansia muciniphila* (AKK P2261) from day 5 to day 14.

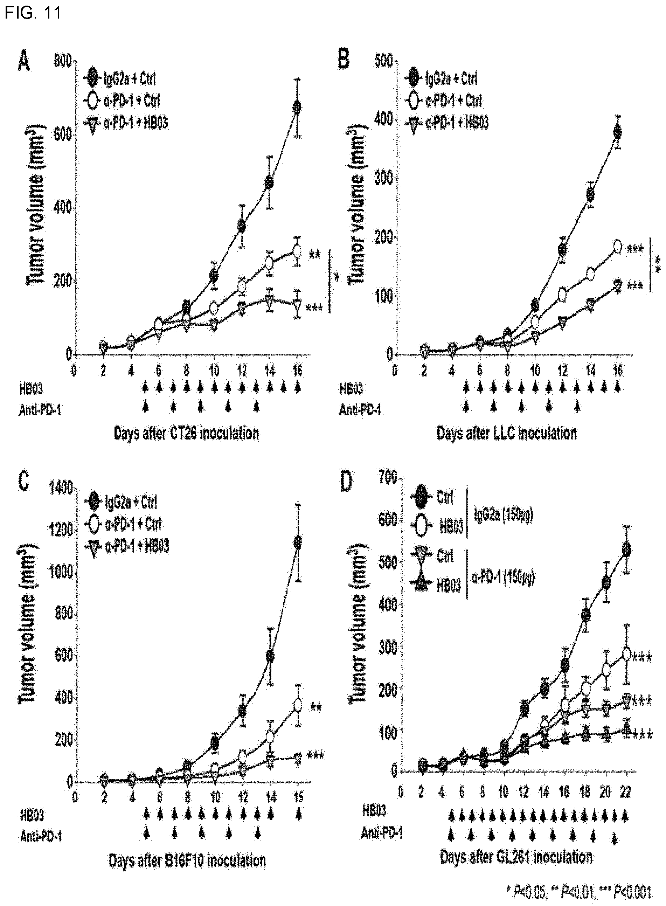

FIG. 11 shows a result of treating mice bearing tumors with an immune checkpoint inhibitor (aPD-1 mAb) and HB03 either alone or in combination and analyzing the difference in tumor growth. (A) Tumor growth kinetics was observed after inoculating BALB/c mice with CT26 colorectal cancer cells and administering mlgG or aPD-1 mAb 5 times until day 16 with 2-day intervals while administering a control substance or HB03 every day from day 5. (B) Tumor growth kinetics was observed after inoculating C57BL/6 JAX mice with LLC lung cancer cells and administering mlgG or aPD-1 mAb 5 times until day 16 with 2-day intervals while administering a control substance or HB03 every day from day 5. (C) Tumor growth kinetics was observed after inoculating C57BL/6 JAX mice with B16F10 melanoma cells and administering mlgG or aPD-1 mAb 5 times until day 15 with 2-day intervals while administering a control substance or HB03 every day from day 5. (D) Tumor growth kinetics was observed after inoculating C57BL/6 JAX mice with GL261 glioma cells and administering mlgG or aPD-1 mAb 5 times until day 15 with 2-day intervals while administering a control substance or HB03 every day from day 5.

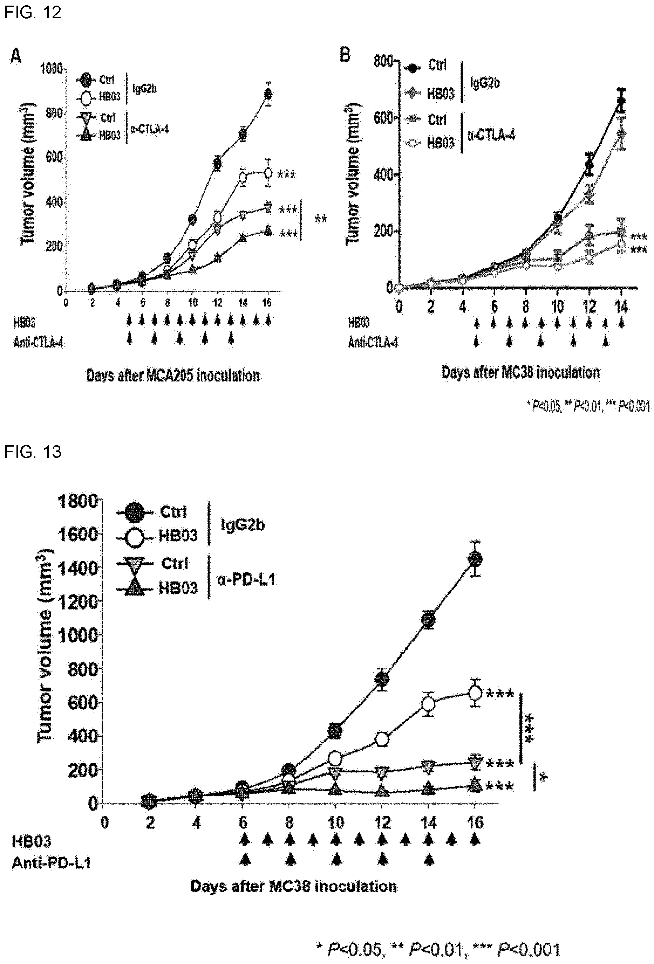

FIG. 12 shows a result of treating mice bearing tumors with an immune checkpoint inhibitor (aCTLA-4 mAb) and HB03 either alone or in combination and analyzing the difference in tumor growth. (A) Tumor growth kinetics was observed after inoculating C57BL/6 JAX mice with MCA205 sarcoma cells and administering mlgG or aCTLA-4 mAb 5 times until day 15 with 2-day intervals while administering a control substance or HB03 every day from day 5. (B) Tumor growth kinetics was observed after inoculating C57BL/6 JAX mice with MC38 colorectal cancer cells and administering mlgG or aCTLA-4 mAb 5 times until day 15 with 2-day intervals while administering a control substance or HB03 every day from day 5.
FIG. 13 shows a result of treating mice bearing tumors with an immune checkpoint inhibitor (aPD-L1 mAb) and HB03 either alone or in combination and analyzing the difference in tumor growth. Tumor growth kinetics was observed after inoculating C57BL/6 JAX mice with MC38 colorectal cancer cells and administering mlgG or aPD-L1 mAb 5 times until day 15 with 2-day intervals while administering a control substance or HB03 every day from day 5.

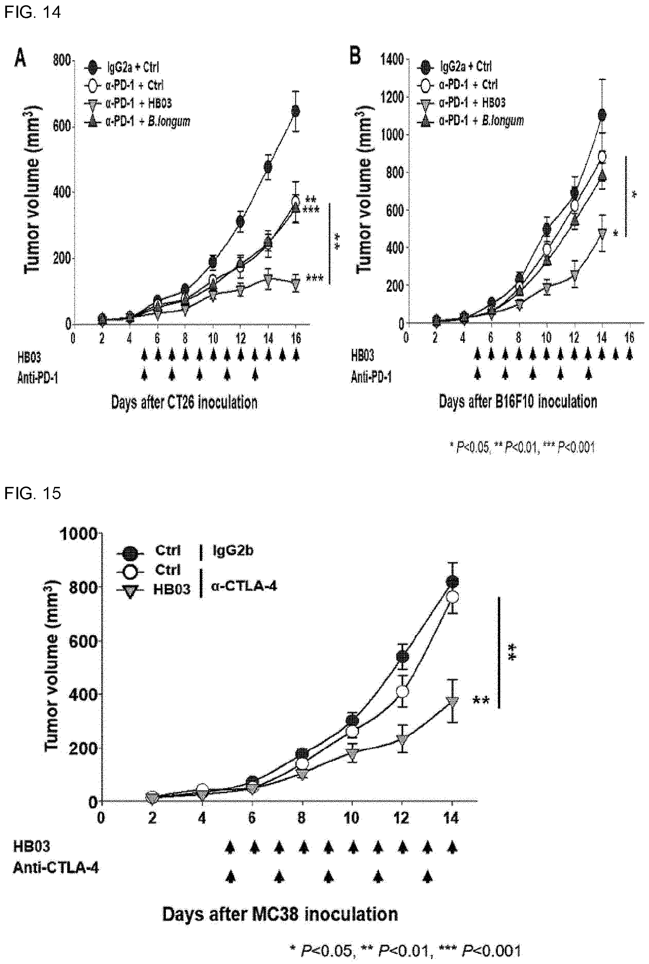

FIG. 14 shows a result of treating mice having resistance to an immune checkpoint inhibitor (aPD-1 mAb) due to antibiotic administration with an immune checkpoint inhibitor (aPD-1 mAb), HB03 and *Bifidobacterium longum* either alone or in combination and analyzing the difference in tumor growth. (A) From 2 weeks before inoculating BALB/c mice with CT26 colorectal cancer cells, the mice were fed with an antibiotic together with water. Only water was given from day 3 after the inoculation and, 48 hours later (from day 5 after the inoculation), mlgG or aPD-1 mAb was administered 5 times until day 16 with 2-day intervals while administering a control substance or HB03 every day. Then, tumor growth kinetics was observed. (B) From 2 weeks before inoculating C57BL/6 JAX mice with B16F10 melanoma cells, the mice were fed with an antibiotic together with water. Only water was given from day 3 after the inoculation and, 48 hours later (from day 5 after the inoculation), mlgG or aPD-1 mAb was administered 5 times until day 16 with 2-day intervals while administering a control substance or HB03 every day. Then, tumor growth kinetics was observed.
FIG. 15 shows a result of treating mice having resistance to an immune checkpoint inhibitor (aCTLA-4 mAb) due to antibiotic administration with an immune checkpoint inhibitor (aCTLA-4 mAb) and HB03 either alone or in combination and analyzing the difference in tumor growth. From 2 weeks before inoculating C57BL/6 JAX mice with MC38 colorectal cancer cells, the mice were fed with an antibiotic together with water. Only water was given from day 3 after the inoculation and, 48 hours later (from day 5 after the inoculation), mlgG or aCTLA-4 mAb was administered 5 times until day 16 with 2-day intervals while administering a control substance or HB03 every day. Then, tumor growth kinetics was observed.

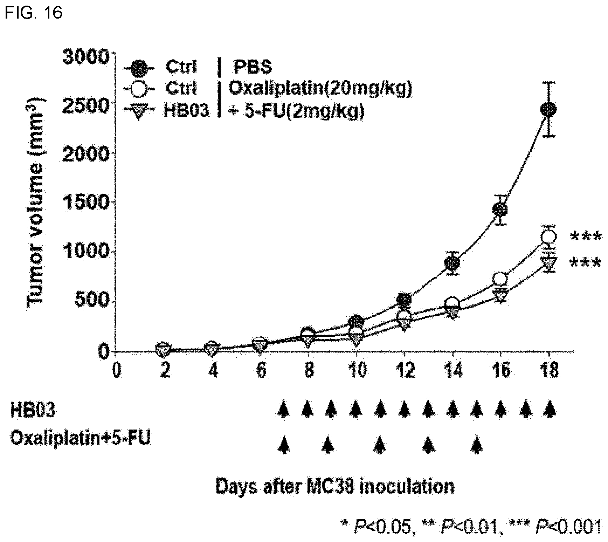

FIG. 16 shows a result of treating mice bearing tumors with an anti-cancer agent (5-FU (5-fluorouracil) or oxaliplatin) and HB03 either alone or in combination and analyzing the difference in tumor growth. Tumor growth kinetics was observed after inoculating C57BL/6 JAX mice with MC38 colorectal cancer cells and then administering PBS, 5-FU or oxaliplatin 5 times until day 16 with 2-day intervals while administering while administering a control substance or HB03 every day from day 5.

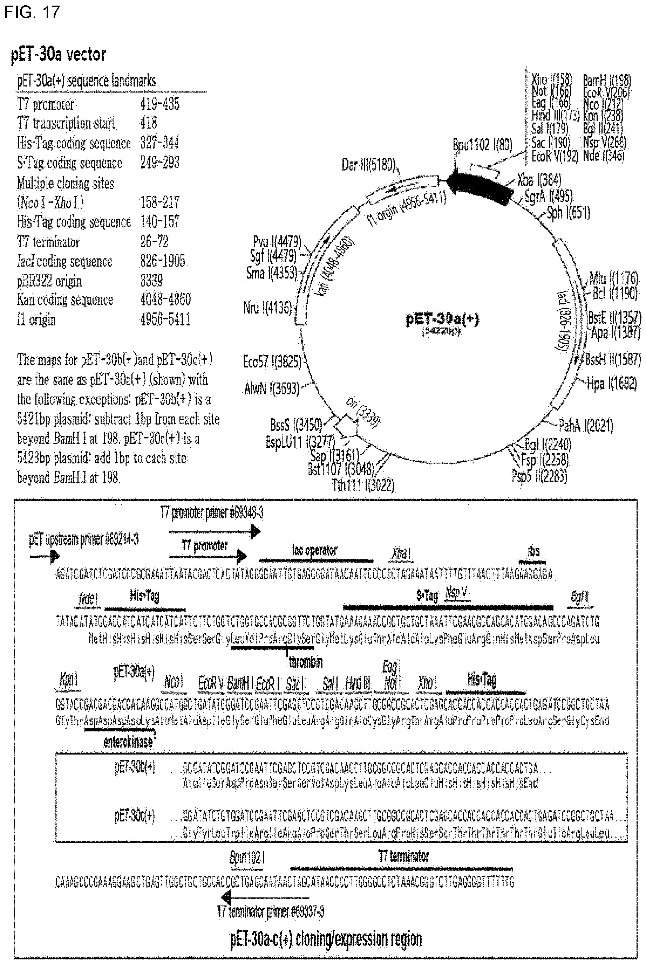

FIG. 17 shows a pET-30a vector.

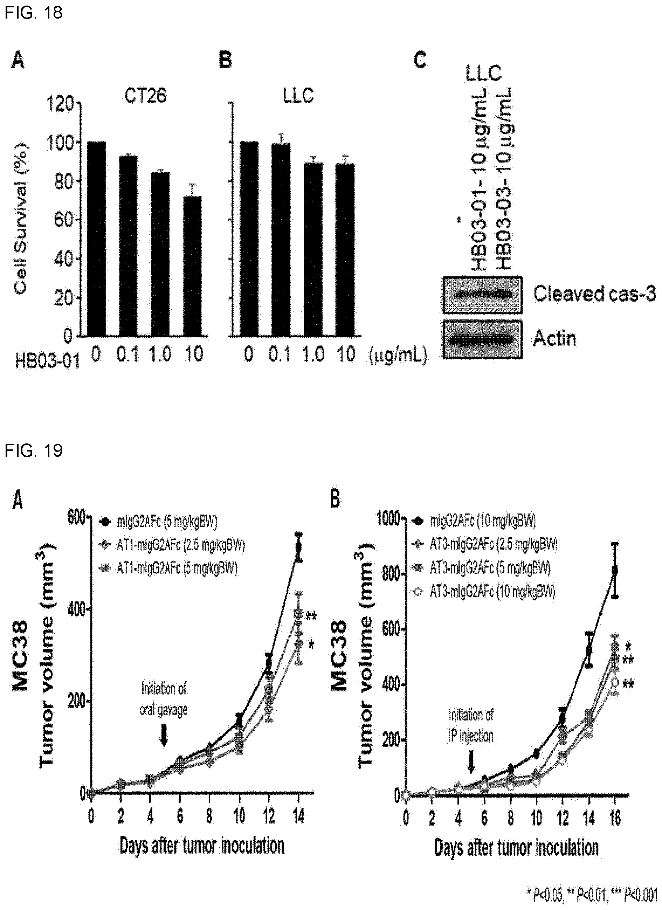

FIG. 18 shows a result of treating tumor cells with proteins secreted by HB03 and analyzing tumor growth and apoptosis. Cell survivability was analyzed after treating CT26 colorectal cancer cells (A) and LLC lung cancer cells (B) with HB03-01, which is a protein derived from HB03, at different concentrations for 48 hours. (C) After treating LLC lung cancer cells with HB03-01 or HB03-03, which are proteins derived from HB03, for 24 hours, it was investigated whether apoptosis was induced based on the activation of caspase-3, which is an apoptosis-regulating protein.
FIG. 19 shows a result of treating mice bearing tumors with proteins secreted by HB03 and analyzing the difference in tumor growth with a control group. (A) Tumor growth kinetics was observed after inoculating C57BL/6 JAX mice with MC38 colorectal cancer cells and then administering a control substance or HB03-01 (AT1) 5 times until day 16 with 2-day intervals from day 5. (B) Tumor growth kinetics was observed after inoculating C57BL/6 JAX mice with MC38 colorectal cancer cells and then administering a control substance or HB03-03 (AT3) 5 times until day 16 with 2-day intervals from day 5.

### [Best Mode]

Hereinafter, the present disclosure is described specifically. The description and exemplary embodiments disclosed in the present disclosure may also be applied to other description and exemplary embodiments. That is to say, all combinations of the various elements disclosed in the present disclosure are included in the scope of the present disclosure. In addition, the scope of the present disclosure is not limited by the following description.

In an aspect, the present disclosure provides a novel *Akkermansia* HB03 strain *(Akkermansia* sp. HB03) deposited with the accession number KCCM12318P.

In the present disclosure, the term *"Akkermansia* strain" refers to a type of bacterium degrading the intestinal mucin of human or animals, which is Gram-negative and obligately (strictly) anaerobic. The *Akkermansia* strain can use mucin as a sole carbon and nitrogen source and can be cultured under a strict anaerobic condition in a medium containing intestinal mucin. In addition, it can proliferate (colonize) in the gastrointestinal tract of various animal species.

The inventors of the present disclosure have isolated a novel *Akkermansia* strain *(Akkermansia* sp.) which is an intestinal mucin-degrading bacterium from the fecal samples of healthy Koreans and have named it an *Akkermansia* HB03 strain *(Akkermansia* sp. HB03). In an example of the present disclosure, in order to investigate whether the isolated *Akkermansia* strain is a novel strain, the genome of the isolated strain was compared with the genomes of previously known *Akkermansia* strains. Specifically, the genome base sequences of a total of 66 *Akkermansia* strains *(Akkermansia* sp.) were searched and clustering was conducted based on the average nucleotide identity of MASH genome base sequences (FIG. 1) (Matthew R Olm et al. dRep: a tool for fast and accurate genome comparisons that enables improved genome recovery from metagenomes through de-replication. ISME J. 2017 Dec; 11(12): 2864-2868.). Subsequent analysis was performed only for the 44 strains belonging to a single species cluster (cluster 3). Roary, which is a high-speed stand-alone pan genome pipeline that takes annotated assemblies in GFF3 format produced by Prokka (Seemann T. Prokka: rapid prokaryotic genome annotation. Bioinformatics. 2014 Jul 15; 30(14): 2068-9.) and calculates a pan genome, was used. Then, an approximately maximum-likelihood tree was constructed using FastTree2 (Price MN, Dehal PS, Arkin AP. FastTree 2--approximately maximum-likelihood trees for large alignments. PLoS One. 2010 Mar 10; 5(3): e9490.) (FIG. 2). 120 strain-specific genes were extracted from a gene presence/absence plot (FIG. 3), and the strain specific for the genes was named an *"Akkermansia* HB03 strain *(Akkermansia* sp. HB03)". As a result of investigating whether a pathogenicity island (PAI), which is a population of genes necessary for exhibiting pathogenicity (toxicity), exists in the whole-genome genes of the HB03 strain, it was confirmed that no pathogenicity island exists in the HB03 strain (FIG. 5). The *"Akkermansia* HB03 strain *(Akkermansia* sp. HB03)" was deposited in the Korean Culture Center of Microorganisms on September 11, 2018 with the accession number KCCM12318P.

The novel *Akkermansia* strain provided by the inventors of the present disclosure has neither been reported yet nor deposited in agencies for preservation and distribution of microorganism resources as general strain or a patented strain. It was first isolated from the feces of healthy Koreans and deposited as or a patented strain by the inventors of the present disclosure.

It was confirmed that the novel *Akkermansia* HB03 strain exhibits a cell yield of 2 times or higher in a modified BTTM medium of the present disclosure as compared to *Akkermansia muciniphila* (Table 1).

In particular, whereas *Akkermansia muciniphila* has the problem that centrifugation and large-scale production are difficult because it tends to suspend rather than being sedimented, the HB03 strain allows easier large-scale production and genome editing than its type strain because of easy centrifugation (FIG. 6). In addition, the HB03 strain lacks pathogenic (toxic) genes and thus does not cause side effects in vivo. Furthermore, it was confirmed that, whereas *Akkermansia muciniphila* requires L-threonine, which is a degradation product of mucin, for growth in a mucin-free medium, the HB03 strain can grow to a concentration of 1.32×10⁹ cells/mL in a medium lacking L-threonine (Table 2).

This suggests that the HB03 strain can grow by using various carbon sources and nitrogen sources other than mucin or L-threonine even unlike the *Akkermansia muciniphila* type strain.

Because the novel *Akkermansia* HB03 strain exhibits superior anti-cancer activity, the novel *Akkermansia* strain, an inactivated strain thereof, a culture thereof, a vesicle thereof, a proteome thereof, a metabolome thereof, etc. can be used as an active ingredient for preventing or treating cancer. This will be described in detail later.

For example, the microorganism of the present disclosure may be one that has been "substantially purified". The expression "substantially purified" refers to a strain or a mixture of strains that has been substantially concentrated from a sample. The microorganism of the present disclosure in the sample may be substantially purified by concentrating to 50%, 60%, 70%, 80%, 90%, 95% or higher. In addition, the sample may contain 50%, 40%, 20%, 15%, 1% or less of microorganisms that lack the effect of the microorganism of the present disclosure or have undesirable effects, although not being limited thereto.

For example, the microorganism contained in the composition of the present disclosure may be encapsulated by coating for oral administration. The coating may be one which prevents the microorganism from being degraded in the gut, although not being limited thereto.

For example, the microorganism of the present disclosure or a culture, vesicle, proteome or metabolome thereof may be one that has been isolated. The term "isolated" refers to a substance which exists in a naturally non-occurring environment or does not exist naturally. It includes substantial isolation from a substance which is naturally associated and found in nature. The isolated substance may be, for example, a substance from which one or more substance associated in natural state has been removed, with its form modified or with its amount changed, although not being limited thereto.

In another aspect, the present disclosure provides a pharmaceutical composition for preventing or treating cancer, which contains one or more selected from a group consisting of a novel *Akkermansia* HB03 strain, an inactivated strain thereof, a culture thereof, a vesicle thereof, a proteome thereof and a metabolome thereof.

The terms used herein are the same as described above.

In the present disclosure, the term "cancer" includes solid cancer and blood cancer, without limitation. The cancer includes various types of cancer, e.g., the cancer of the head, neck, eye, mouth, throat, esophagus, chest, bone, lung, colon, rectum, stomach, prostate, breast, ovary, kidney, bladder, liver, pancreas or brain, without limitation. The cancer may include primary and metastatic cancer unless specified otherwise. The cancer may include, for example, lung cancer, pancreatic cancer, stomach cancer, liver cancer, colorectal cancer, brain cancer, breast cancer, thyroid cancer, bladder cancer, esophageal cancer, leukemia, ovarian cancer, melanoma, head and neck cancer, skin cancer, prostate cancer, colorectal cancer, hepatocellular carcinoma, fibrosarcoma or cervical cancer.

For the purpose of the present disclosure, the cancer may include cancer which exhibits resistance to an anti-cancer agent, although not being limited thereto.

The anti-cancer agent may include a chemical anti-cancer agent, a targeted anti-cancer agent or an immune checkpoint inhibitor, although not being limited thereto.

In the present disclosure, the term "chemical anti-cancer agent" refers to a therapeutic agent developed to inhibit the proliferation of cancer cells. The mechanism of action varies depending on the anti-cancer agent, e.g., inhibition of DNA synthesis, etc., and it is used as a major therapeutic tool for cancer to which the anti-cancer agent responds well, such as acute leukemia, lymphoma, testicular cancer, etc. The chemical anti-cancer agent is also called a cytotoxic anti-cancer agent. Examples of the chemical anti-cancer agent include gemcitabine, cytarabine, carboplatin (Paraplatin), cisplatin (Platinol, Platinol-AQ), crizotinib (Xalkori), cyclophosphamide (Cytoxan, Neosar), docetaxel (Taxotere), doxorubicin (Adriamycin), erlotinib (Tarceva), etoposide (Vepesid), 5-fluorouracil (5-FU), irinotecan (Camptosar), liposome-encapsulated doxorubicin (Doxil), methotrexate (Forlex, Mexate, amethopterin), paclitaxel (Taxol, Abraxane), topotecan (Hycamtin), trabectedin (Yondelis), vincristine (Oncovin, Vincasar PFS), vinblastine (Velban), etc.

In the present disclosure, the term "targeted anti-cancer agent" refers to an anti-cancer agent which attacks cancer cells only by recognizing specific markers they possess without harming normal cells. It exhibits several times higher killing ability for cancer cells. Examples of the targeted anti-cancer agent include imatinib mesylate (Gleevec), cetuximab (Erbitux), gefitinib (Iressa), olmutinib (Olita), osimertinib (Tagrisso), bevacizumab (Avastin), sorafenib (Nexavar), oxaliplatin (Eloxatin), sunitinib (Sutent), etc.

In the present disclosure, the term "immune checkpoint inhibitor" refers to an antibody therapeutic agent which emerges as a 3rd-generation anti-cancer agent following the 1st-generation anti-cancer agent for surgery, chemotherapy (chemical anti-cancer agent) and radiation therapy and the targeted therapy (2nd-generation anti-cancer agent or targeted anti-cancer agent). It is advantageous in that it exhibits powerful anti-cancer effect by activating the immune function of immune cells and is applicable to various indications. In addition, it has few side effects because it functions for a given period of time only to prevent damage of normal cells due to excessive immune activity. Examples of the immune checkpoint inhibitor include PD-1 (programmed cell death protein 1), CTLA4 (cytotoxic T-lymphocyte-associated antigen 4), PD-L1 (programmed death-ligand 1), PD-L2 (programmed death-ligand 2), A2AR (adenosine A2A receptor), B7-J3 (B7 family molecule), B7-H4 (B7 family molecule), BTLA (B- and T-lymphocyte attenuator), Kirs (killer cell immunoglobulin-like receptors), LAG3 (lymphocyte-activation gene 3), TIM-3 (T-cell immunoglobulin and mucin-domain containing-3, HAVCR2 (hepatitis A virus cellular receptor 2), VISTA (V-domain Ig suppressor of T cell activation), etc.

The inhibitor may be a monoclonal antibody (mAb) targeting the substance described above, e.g., an antibody against PD-1 (αPD-1 mAb), an antibody against CTLA-4 (αCTLA-4 mAb) or an antibody against PD-L1 (αPD-L1 mAb), although not being limited thereto.

In the present disclosure, the term "prevention" refers to any action of suppressing or delaying the onset of a disease by administering the pharmaceutical composition according to the present disclosure, and the term "treatment" refers to any action of improving or favorably changing the symptoms of a disease by administering the pharmaceutical composition.

The 'prevention or treatment of cancer' may mean an 'anticancer' effect.

In the present disclosure, the term "anticancer" includes the prevention, treatment, amelioration of symptoms and alleviation of cancer.

In the present disclosure, the term "anti-tumor" means the reduction in size or elimination of malignant tumor occurring from solid cancer.

In the present disclosure, the term "inactivated strain" refers to a strain which has been non-functionalized or killed. The method for non-functionalizing or killing a strain includes thermal inactivation, pH inactivation, chemical inactivation, etc. The inactivated strain may include a cell lysate or an extracellular product derived from the non-functionalized or killed strain.

In the present disclosure, the inactivation may be thermal inactivation by heating, although not being limited thereto.

In the present disclosure, the term "culture" may include a culture itself obtained by culturing a strain, or a concentrate or lyophilization product of a supernatant obtained by removing the strain from the culture.

In the present disclosure, the culture refers to a product obtained by culturing the novel *Akkermansia* HB03 strain. In a broad sense, the culture may be the whole culture of the *Akkermansia* strain, a supernatant of the culture, a lysate of the culture, a fraction thereof, etc. The supernatant may be obtained by centrifuging the culture of the strain, the lysate may be obtained by physical treatment or sonication of the strain, and the fraction may be obtained by conducting centrifugation, chromatography, etc. on the culture, supernatant, lysate, etc.

In the present disclosure, the term "culturing" refers to a series of action of growing a microorganism under an artificially controlled appropriate condition.

In the present disclosure, the culturing may be understood to mean a method of culturing the *Akkermansia* strain provided in the present disclosure. The culturing may be performed using methods widely known in the art. Specifically, the culturing may be performed continuously by a batch, fed-batch or repeated fed-batch process.

The *Akkermansia* strain should be cultured in a medium containing mucin, suitable carbon source and nitrogen source, amino acids, vitamins, etc. under an anaerobic condition satisfying the requirements for the specific strain such as temperature, pH, etc. As the carbon source, mucin or a mixture with N-acetylglucosamine, glucose and xylose may be used. In addition, a sugar or a carbohydrate such as galactosamine, sucrose, lactose, fructose, maltose, starch and cellulose, an oil or fat such as soybean oil, sunflower oil, castor oil, coconut oil, etc., a fatty acid such as palmitic acid, stearic acid and linoleic acid, an alcohol such as glycerol and ethanol, and an organic acid such as acetic acid, may be used. These substances may be used individually or in admixture. As the nitrogen source, an inorganic nitrogen source such as ammonia, ammonium sulfate, ammonium chloride, ammonium acetate, ammonium phosphate, ammonium carbonate and ammonium nitrate; or an organic nitrogen source such as an amino acid such as glutamic acid, methionine and glutamine, soytone, tryptone, peptone, NZ-amine, meat extract, yeast extract, malt extract, corn steep liquor, casein hydrolysate, fish or a degradation product thereof, defatted soybean cake or a degradation product thereof, etc. may be used. These nitrogen sources may be used either alone or in combination. The medium may contain monopotassium phosphate, dipotassium phosphate or a sodium-containing salt thereof as a phosphorus source. The phosphorus source includes potassium dihydrogen phosphate, potassium phosphate dibasic or a sodium-containing salt thereof. In addition, an inorganic compound such as sodium chloride, calcium chloride, iron chloride, magnesium sulfate, iron sulfate, manganese sulfate, calcium carbonate, etc. may be used. Finally, a substance essential for growth such as an amino acid and a vitamin may be used. In addition, a defoamer such as a fatty acid polyglycol ester may be used to prevent foaming.

In an example of the present disclosure, the *Akkermansia* HB03 strain of the present disclosure and the *Akkermansia muciniphila* type strain (ATCC BAA-835) were cultured using a modified BTTM medium with temperature maintained at 37 °C, although not being limited thereto.

In the present disclosure, the term "vesicle" refers to a structure present in a cell, which is in the form of a relatively small sack and encloses and transports a chemical substance.

In the present disclosure, the term "proteome" refers to the entire set of proteins expressed by various biological entities such as a specific cell, tissue, organism, etc.

In the present disclosure, the proteome may be a protein derived from a novel *Akkermansia* strain, e.g., HB03-01, HB03-02 or HB03-03, although not being limited thereto.

In the present disclosure, the term "metabolome" refers to the complete set of metabolites existing in a biological sample such as a cell, tissue, body fluid, etc. In general, it consists of substances of 1500 daltons or smaller in size, and is classified into an endogenous metabolome (amino acids, nucleic acid, fatty acid, sugars, amines, glycolipids, short peptides, vitamins, hormones, etc.) and an exogenous metabolome (e.g., drugs, foods, additives, toxic substances, etc.). Often, target substances produced from degradation of DNAs, RNAs or proteins are also classified as a metabolome. Because the metabolome changes quickly and dynamically every second as a result of metabolism, it immediately reflects the change in phenotype due to drug response or food intake as compared to a genome or a proteome (Korean Society for Biochemistry and Molecular Biology).

The HB03-01 may consist of an amino acid sequence of SEQ ID NO 1, and the HB03-03 may consist of an amino acid sequence of SEQ ID NO 2.

Although the HB03-01 or HB03-03 protein is defined in the present disclosure as consisting of an amino acid sequence of SEQ ID NO 1 or SEQ ID NO 2, mutation that may occur by addition of an insignificant sequence before or after the amino acid sequence of SEQ ID NO 1 or SEQ ID NO 2 or naturally occurring mutation or silent mutation thereof is not excluded. Also, it is obvious to those skilled in the art that a protein having the same or comparable activity as the polypeptide including an amino acid sequence of SEQ ID NO 1 or SEQ ID NO 2 corresponds to the protein of the present disclosure. As a specific example, the protein of the present disclosure may be a protein consisting of an amino acid sequence having 80%, 85%, 90%, 95%, 96%, 97%, 98%, 99% or higher homology or identity to the amino acid sequence of SEQ ID NO 1 or SEQ ID NO 2. In addition, it is obvious that a polypeptide consisting of an amino acid sequence having such homology or identity and exhibiting efficiency comparable to that of the protein, wherein some of the sequence has been deleted, modified, substituted or added, is also included in the scope of the protein of the present disclosure.

That is to say, it is obvious that, even if 'a polypeptide consisting of an amino acid sequence of a specific sequence number' is described in the present disclosure, a polypeptide consisting of an amino acid sequence exhibiting efficiency comparable to that of the polypeptide consisting of an amino acid sequence of the specific sequence number, wherein some of the sequence has been deleted, modified, substituted or added, may be used if it has the same or comparable activity. For example, 'a polypeptide consisting of an amino acid sequence of SEQ ID NO 1' may include a polypeptide having the same or comparable activity to that of the 'polypeptide consisting of an amino acid sequence of SEQ ID NO 1'.

The polypeptide may be conjugated with another sequence or a linker for identification, purification or synthesis of the polypeptide.

The pharmaceutical composition may contain an extract, fraction, suspension, etc. derived from the novel *Akkermansia* HB03 strain, an inactivated strain thereof, a culture thereof, a vesicle thereof, a proteome thereof and a metabolome thereof without limitation.

In the present disclosure, the term "extract" includes an extract obtained by extracting the novel *Akkermansia* strain provided in the present disclosure, a diluate or concentrate of the extract, a dried product obtained by drying the extract, a crude purification product or a purification product of the extract, a mixture thereof, etc. An extraction method for obtaining the extract is not particularly limited, and the methods commonly used in the art may be used. Nonlimiting examples of the extraction method include hot water extraction, ultrasonic extraction, filtration, reflux extraction, etc., and they may be performed either alone or in combination. An extraction solvent used to obtain the extract is not particularly limited, and any solvent known in the art may be used. Nonlimiting examples of the extraction solvent include water, an alcohol or a mixture solvent thereof, and they may be used either alone or in combination.

In the present disclosure, the term "fraction" refers to a product obtained by conducting fractionation to separate a specific component or a group of specific components from a mixture containing various components. A method for obtaining the fraction is not particularly limited, and the methods commonly used in the art may be used. A fractionation solvent used to obtain the fraction is not particularly limited, and any solvent known in the art may be used. Nonlimiting examples of the fractionation solvent include a polar solvent such as water, an alcohol, etc. and a nonpolar solvent such as hexane, ethyl acetate, chloroform, dichloromethane, etc. They may be used either alone or in combination.

In the present disclosure, the term "suspension" refers to a mixture wherein fine solid particles are dispersed homogeneously in a liquid without being dissolved.

In the present disclosure, the suspension may be one wherein a specified number of lyophilized strains are dispersed in water, PBS (phosphate-buffered saline) or a culture medium (liquid broth). The term "suspension" may be used interchangeably with a "strain composition".

In the present disclosure, the pharmaceutical composition according to the present disclosure may be co-administered with an anti-cancer agent.

In the present disclosure, the term "co-administration" refers to administration of the pharmaceutical composition of the present disclosure together with an anti-cancer agent for treating cancer simultaneously or independently or non-independently (in admixture) with a time interval, for preventing, treating or alleviating or mitigating the symptoms of cancer. Through the co-administration, higher anti-cancer activity may be achieved as compared to single administration of the pharmaceutical composition of the present disclosure or the anti-cancer agent. In addition, higher anti-cancer activity may be achieved for cancer exhibiting resistance to an anti-cancer agent as compared to single administration of the anti-cancer agent.

The anti-cancer agent may be, for example, a chemical anti-cancer agent, a targeted anti-cancer agent or an immune checkpoint inhibitor, although not being limited thereto. The chemical anti-cancer agent, the targeted anti-cancer agent or the immune checkpoint inhibitor are the same as described above. However, any previously known anti-cancer agent may be used without being limited thereto.

In an example of the present disclosure, the co-administration of an immune checkpoint inhibitor (αPD-1 mAb, αCTLA-4 mAb or αPD-L1 mAb) and HB03 to animal models of colorectal cancer (CT26, MC38), lung cancer (LLC), melanoma (B16F10), glioma (GL261) and sarcoma (MCA205) resulted in significantly increased anti-tumor effect as compared to when they were treated with the immune checkpoint inhibitor only (FIGS. 11-13).

Also, in another example of the present disclosure, the co-administration of an anti-cancer agent (5-FU (5-fluorouracil), oxaliplatin) and HB03 to an animal model of colorectal cancer (MC38) resulted in significantly increased anti-tumor effect as compared to single administration of the anti-cancer agent (FIG. 16).

These results suggest that the novel *Akkermansia* strain of the present disclosure effectively inhibits tumorigenesis with its anti-cancer activity while reducing the administration dosage of the anti-cancer agent.

In the present disclosure, the pharmaceutical composition according to the present disclosure may be co-administered to an individual which exhibits resistance to an anti-cancer agent.

The anti-cancer agent may include an immune checkpoint inhibitor, a chemical anti-cancer agent or a targeted anti-cancer agent, although not being limited thereto.

The immune checkpoint inhibitor is the same as described above. 70% or more of patients with cancer do not respond to the immune checkpoint inhibitor or resistance is induced.

In an example of the present disclosure, when an immune checkpoint inhibitor (αPD-1 mAb or αCTLA-4 mAb) and HB03 were co-administered to animal models of colorectal cancer (CT26, MC38) and melanoma (B16F10) having resistance to the immune checkpoint inhibitor, the co-administration of αPD-1 mAb and HB03 resulted in significantly increased anti-tumor effect as compared to single administration of αPD-1 mAb, and this effect was superior to that when they were co-administered the previously known *Akkermansia muciniphila* strain or *Bifidobacterium longum* strain (FIG. 14). In addition, the co-administration of αCTLA-4 mAb and HB03 resulted in significantly increased anti-tumor effect as compared to single administration of αCTLA-4 mAb (FIG. 15).

This result suggests that the co-administered of the novel *Akkermansia* strain of the present disclosure effectively inhibits tumorigenesis even for the cancer exhibiting resistance to an immune checkpoint inhibitor owing to its anti-cancer activity.

The pharmaceutical composition of the present disclosure may contain the extract in an amount of 0.0001-50 wt%, specifically 0.01 wt%-10 wt%, based on the total weight of the composition, although not being limited thereto.

The pharmaceutical composition according to the present disclosure may further contain an adequate carrier, excipient or diluent commonly used for preparation of a pharmaceutical composition. Specifically, the pharmaceutical composition may be formulated into an oral formulation such as a powder, a granule, a tablet, a capsule, a suspension, an emulsion, a syrup, an aerosol, etc., a formulation for external application, a suppository or a sterilized injection solution according to common methods. In the present disclosure, the carrier, excipient or diluent that may be contained in the pharmaceutical composition may be lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate and mineral oil. The formulation is prepared by a using commonly diluent or excipient such as a filler, an extender, a binder, a wetting agent, a disintegrant, a surfactant, etc. Solid formulations for oral administration include a tablet, a pill, a powder, a granule, a capsule, etc., and the solid formulation is prepared by mixing the extract or a fraction thereof with at least one excipient, e.g., starch, calcium carbonate, sucrose, lactose, gelatin, etc. In addition to a simple excipient, a lubricant such as magnesium stearate and talc is also used. Liquid formulations for oral administration include a suspension, an internal solution, an emulsion, a syrup, etc. In addition to a commonly used simple diluent such as water or liquid paraffin, various excipients, e.g., a wetting agent, a sweetener, an aromatic, a preservative, etc. may be contained. Formulations for parenteral administration include a sterilized aqueous solution, a nonaqueous solution, a suspension, an emulsion, a lyophilized formulation and a suppository. For the nonaqueous solution or suspension, propylene glycol, polyethylene glycol, a vegetable oil such as olive oil, an injectable ester such as ethyl oleate, etc. may be used. As a base of the suppository, witepsol, macrogol, Tween 61, cocoa butter, laurin butter, glycerogelatin, etc. may be used.

In an example of the present disclosure, as a result of pre-treating (administration before tumor inoculation) or post-treating (administration after tumor inoculation) a strain composition HB03 prepared by resuspending the lyophilized novel *Akkermansia* HB03 strain in a liquid culture medium or HB03 (P) prepared by thermally inactivating the HB03 to tumor animal models of colorectal cancer (CT26), lung cancer (LLC), pancreatic cancer (Panc02), melanoma (B16F10), glioma (GL261) and sarcoma (MCA205) for a predetermined period, tumor growth was decreased remarkably (FIG. 7 and FIG. 9), and this anti-tumor effect was superior to that when a *Akkermansia muciniphila* type strain (ATCC BAA-835 strain), or a *Bifidobacterium longum* strain (ATCC BAA-999 strain) was administered (FIG. 8). And, when the anti-cancer effect was compared after administration of AKK p2261 or HB03, the administration of HB03 increased the anti-cancer effect further(FIG. 10),. Through this, it was confirmed that HB03 has superior anti-cancer effect as compared to other 32 strains.

In another example of the present disclosure, HB03-01, which is a protein derived from HB03, significantly decreased the proliferation of colorectal cancer and lung cancer cells (FIGS. 18A and 18B) and activated the apoptosis-regulating enzyme (caspase-3) of lung cancer cells together with HB03-03, which is another protein (FIG. 18C). This result suggests that the novel *Akkermansia* strain of the present disclosure can be used to prevent or treat cancer owing to its anti-cancer activity.

In another aspect, the present disclosure provides a method for preventing or treating cancer, which includes a step of administering the pharmaceutical composition to an individual.

The terms used hereinafter are the same as described above.

In the present disclosure, the term "individual" refers to any animal which shows or has the risk of showing the symptoms of cancer. An individual suspected to have cancer may be treated effectively by administering the pharmaceutical composition of the present disclosure to the individual. The pharmaceutical composition of the present disclosure may be applied to any individual for the purpose of preventing or treating cancer, without special limitation. For example, the individual may be human, non-human mammals such as monkey, dog, cat, rabbit, guinea pig, rat, mouse, cow, sheep, pig, goat, etc., birds, fish, etc. In addition, it may be a non-human animal, although not being limited thereto.

In the present disclosure, the term "administration" refers to introduction of a substance into an individual by a suitable method.

The pharmaceutical composition of the present disclosure may be administered in a pharmaceutically effective amount. In the present disclosure, the term "pharmaceutically effective amount" refers to an amount sufficient to treat or prevent a disease at a reasonable benefit/risk ratio applicable to medical treatment or prevention. An effective dosage level may be determined depending on the severity of the disease, the activity of a drug, the age, body weight, health condition and sex of a patient, the patient's sensitivity to the drug, administration time, administration route, excretion rate, the duration of treatment, drugs used concurrently in combination with the composition of the present disclosure, and other factors well known in the medical field. The pharmaceutical composition of the present disclosure may be administered as an independent therapeutic agent or in combination with another therapeutic agent, and may be administered sequentially or simultaneously with another therapeutic agent. It may also be administered as a single or multiple dosage form. It is important to administer the composition in the minimum amount that can exhibit the maximum effect without causing side effects, in consideration of all the above-described factors.

Specifically, the administration dosage of the pharmaceutical composition according to the present disclosure may be, for example, about 0.0001-100 mg/kg, specifically 0.001-10 mg/kg, per day, for a mammal. The daily dosage of the pharmaceutical composition of the present disclosure may be administered once or several times a day, although not being specially limited thereto. The above-described administration dosage does not limit the scope of the present disclosure by any means.

The administration route or administration mode of the pharmaceutical composition of the present disclosure is not specially limited, and any administration route or administration mode may be employed as long as the composition can be can be delivered to the desired target site. Specifically, the composition may be administered via oral or various parenteral routes. Nonlimiting examples of the administration route include oral, rectal, topical, intravenous, intraperitoneal, intramuscular, intraarterial, transdermal or intranasal routes. For topical treatment, it may be administered by a suitable method including intralesional administration, if necessary. In addition, the pharmaceutical composition may be administered by any device capable of delivering an active substance to a target cell.

In another aspect, the present disclosure provides a food composition for preventing or alleviating cancer, which contains one or more selected from a group consisting of the novel *Akkermansia* HB03 strain, an inactivated strain thereof, a culture thereof, a vesicle thereof, a proteome thereof and a metabolome thereof.

The terms used herein are the same as described above.

In the present disclosure, the term "alleviation" refers to any action of improving or favorably changing the symptoms of a disease by administering the composition. In the present disclosure, the disease refers to cancer.

In the present disclosure, the term "food" includes any food in ordinary meaning, such as meat, sausage, bread, chocolate, candies, snacks, confectionery, pizza, ramen, other noodles, gums, dairy products including ice creams, soups, beverages, tea, drinks, alcoholic beverages, vitamin complexes, functional health foods, health foods, etc.

The functional health food is the same term as a food for special health use (FoSHU), and means a food having high medical effect, which is processed to effectively exhibit bioregulatory function in addition nutrition. Here, the term "functional" means obtainment of an effect useful for regulation of a nutrient with respect to the structure and function of the human body or hygienic use such as physiological action, etc. The food of the present disclosure may be prepared by a method commonly used in the art, and may be prepared by adding a raw material and ingredients commonly used in the art. In addition, the food may be prepared into any formulation that is recognized as food, without limitation. The food composition of the present disclosure may be prepared into various formulations, and is advantageous in that it has no side effect that may occur during long-term use because it contains food materials unlike general drugs and has excellent portability. Therefore, the food of the present disclosure can be taken as a supplement to enhance the effect of preventing or alleviating cancer.

The health food refers to a food having an active effect of maintaining or improving health as compared to common food, and means a health supplement food or a food intended for health supplement. In some cases, the terms functional health food, health food and health supplement food can be used interchangeably.

Specifically, the functional health food is a food prepared by adding the composition of the present disclosure to a food material such as a beverage, tea, a spice, gum, confectionery, etc. or prepared into a capsule, a powder, a suspension, etc. When ingested, it provides a special effect on health. But, since it contains a food as a raw material unlike general drugs, it is advantageous in that it has no side effect that may occur during long-term use of drugs.

The food composition of the present disclosure can be used very usefully because it can be taken routinely and, thus, high effect of preventing or alleviating cancer can be expected.

The food composition may further contain a physiologically acceptable carrier. The type of the carrier is not particularly limited and any carrier commonly used in the art may be used.

In addition, the food composition may contain an additional ingredient which is commonly contained in a food composition to improve odor, taste, visual appearance, etc. For example, it may contain vitamin A, C, D, E, B₁, B₂, B₆ or B₁₂, niacin, biotin, folate, pantothenic acid, etc. In addition, it may contain a mineral such as zinc (Zn), iron (Fe), calcium (Ca), chromium (Cr), magnesium (Mg), manganese (Mn), copper (Cu), etc. In addition, it may contain an amino acid such as lysine, tryptophan, cysteine, valine, etc.

In addition, the food composition may contain food additives such as a preservative (potassium sorbate, sodium benzoate, salicylic acid, sodium dehydroacetate, etc.), a sterilizer (bleaching powder, high-grade bleaching powder, sodium hypochlorite, etc.), an antioxidant (butylhydroxyanisole (BHA), butylhydroxytoluene (BHT), etc.), a dye (tar pigment, etc.), a colorant (sodium nitride, sodium nitrite, etc.), a bleaching agent (sodium sulfite), a flavor enhancer (MSG, sodium glutamate, etc.), a sweetener (dulcin, cyclamate, saccharin, etc.), a flavorant (vanillin, lactone, etc.), a swelling agent (alum, potassium D-bitartate, etc.), a reinforcing agent, an emulsifier, a thickener, a coating agent, a gum base, a defoamer, a solvent, an improving agent, etc. The additive may be selected according to the type of the food and may be used in an appropriate amount.

The composition of the present disclosure may be added either alone or together with another food or food ingredient, according to common methods. The mixing amount of the active ingredient may be determined appropriately depending on the purpose of use (prevention, health care or therapeutic treatment). In general, for preparation of a food or a beverage, the food composition of the present disclosure may be added in an amount of 50 parts by weight or less, specifically 20 parts by weight or less, based on the food or beverage. However, in case of long-term intake for the purpose of health or hygiene improvement, a smaller amount may be used. And, since there is no safety issue, the active ingredient may be used in a larger amount.

For example, the food composition of the present disclosure may be used as a health beverage composition and, in this case, it may further contain various flavorants, natural carbohydrates, etc. as common beverages. The natural carbohydrate may be a monosaccharide such as glucose or fructose, a disaccharide such as maltose or sucrose, a polysaccharide such as dextrin or cyclodextrin, or a sugar alcohol such as xylitol, sorbitol, erythritol, etc. As a sweetener, a natural sweetener such as thaumatin or stevia extract or a synthetic sweetener such as saccharin or aspartame may be used. The proportion of the natural carbohydrate may be generally about 0.01-0.04 g, specifically about 0.02-0.03 g, per 100 mL of the health beverage composition of the present disclosure.

In addition, the health beverage composition may contain various nutrients, vitamins, electrolyte, flavorants, colorants, pectic acid, salts of pectic acid, alginic acid, salts of alginic acid, organic acids, protective colloidal thickeners, pH control agents, stabilizers, preservatives, glycerin, alcohols, carbonating agents, etc. In addition, it may contain a pulp for preparation of natural fruit juice, fruit juice beverages or vegetable beverages. These ingredients may be used either alone or in combination. The proportion of these additives is of no great importance, but is usually within a range of 0.01-0.1 part by weight per 100 parts by weight of the health beverage composition of the present disclosure.

The content of the active ingredient in the food composition of the present disclosure may vary as long as the effect of preventing or alleviating cancer can be achieved. For example, the strain according to the present disclosure may be contained at a content of 0.00001-100 wt% or 0.01-80 wt% based on the total weight of the food composition, although not being limited thereto.

In another aspect, the present disclosure provides a use of the novel *Akkermansia* HB03 strain *(Akkermansia* sp. HB03) deposited with the accession number KCCM12318P, an inactivated strain thereof, a culture thereof, a vesicle thereof, a proteome thereof or a metabolome thereof for prevention or treatment of cancer.

The terms used herein are the same as described above.

### [Mode for Invention]

Hereinafter, the present disclosure is described more specifically through examples. However, the examples are provided only to illustrate the present disclosure and the scope of the present disclosure is not limited by the examples.

### Example 1: Identification of novel strain through genome base sequencing

A total of 66 *Akkermansia* species were searched from NCBI and clustering was conducted based on the average nucleotide identity (ANI) of MASH genome base sequences using dRep (FIG. 1) (Matthew R Olm et al. dRep: a tool for fast and accurate genome comparisons that enables improved genome recovery from metagenomes through de-replication. ISME J. 2017 Dec; 11(12): 2864-2868.). Subsequent analysis was performed only for the 44 strains belonging to a single species cluster (cluster 3).

Roary, which is a high-speed stand-alone pan genome pipeline that takes annotated assemblies in GFF3 format produced by Prokka (Seemann T. Prokka: rapid prokaryotic genome annotation. Bioinformatics. 2014 Jul 15; 30(14): 2068-2069.) and calculates a pan genome, was used. Then, an approximately maximum-likelihood tree was constructed using FastTree2 (Price MN, Dehal PS, Arkin AP. FastTree 2--approximately maximum-likelihood trees for large alignments. PLoS One. 2010 Mar 10; 5(3): e9490.) (FIG. 2). 120 strain-specific genes were extracted from a gene presence/absence plot (FIG. 3). Most of them were hypothetical proteins whose functions are not known. The strain specific for these genes was named *"Akkermansia* HB03 *(Akkermansia* sp. HB03)".

Because Roary depends on the protein sequence translated from the active CDS (coding sequence), the genome base sequences having errors or defective annotations were excluded from analysis. Therefore, the HB03 strain-specific genes predicted by Roary were searched for genome sequences (not protein coding sequences) and it was confirmed that they are nonexistent. The presence of the genes was investigated by calculating blast score ratio (BSR) using LS-BSR (large-scale blast score ratio) (FIGS. 4a and 4b).

As a result of investigating whether a pathogenicity island (PAI), which is a population of genes necessary for exhibiting pathogenicity (toxicity), exists in the whole-genome genes of the HB03 strain, it was confirmed that no pathogenicity island exists in the HB03 strain (FIG. 5).

As a result of comparing with the whole genome of a *Akkermansia muciniphila* type strain (ATCC BAA-835), the HB03 strain showed a difference of 8%. Even for the genome-matched portion, the ANI value was only 97%, suggesting that the stain is a novel strain distinguished from the type strain.

### Example 2: Culturing characteristics of novel strain

The HB03 strain was cultured in a modified BTTM medium *(*Nat. Med. 2017. 23: 107-113) (BTTM with the content of N-acetylglucosamine decreased to 1/10 and the content of L-threonine decreased to 1/5) at 37 °C under an anaerobic condition.

As a result, as shown in Table 1, the HB03 strain showed 2 times or higher cell yield and 2 times or higher growth rate as compared to *Akkermansia muciniphila.*

**[Table 1]**

| Days (hours) of culturing at 37 °C | 1 day (24 hours) (cells/mL) | 2 days (48 hours) (cells/mL) | 3 days (72 hours) (cells/mL) |
|---|---|---|---|
| ATCC BAA-835^{T} | 5.77 × 10⁸ | 1.97 × 10⁹ | 2.15 × 10⁹ |
| HB03 | 1.3 × 10⁹ | 4.74 × 10⁹ | 5.67 × 10⁹ |

In addition, whereas *Akkermansia muciniphila* has the problem that centrifugation and large-scale production are difficult because it tends to suspend rather than being sedimented, the HB03 strain allowed easier large-scale production and genome editing than its type strain because of easy centrifugation (FIG. 6).

In addition, whereas the *Akkermansia muciniphila* strain did not grow when the amino acid L-threonine was absent in the medium, the HB03 strain grew to a concentration of 1.0×10⁹ cells/mL in a medium lacking L-threonine (Table 2). This means that the HB03 strain can grow even in the absence of mucin or L-threonine, which is a degradation product of mucin, unlike the *Akkermansia muciniphila* type strain, by using various carbon sources and nitrogen sources other than mucin or L-threonine as nutrients.

**[Table 2]**

| Medium composition (37 °C, culturing for 2 days) | ATCC BAA-835 (cells/mL) | HB03 (cells/mL) |
|---|---|---|
| HB medium | 1.97 × 10⁹ | 4.74 × 10⁹ |
| HB medium (excluding glucose) | 1.79 × 10⁹ | 1.63 × 10⁹ |
| HB medium (excluding tryptone) | 1.75 × 10⁹ | 1.02 × 10⁸ |
| HB medium (excluding N-acetylglucosamine) | No growth | No growth |
| HB medium (excluding threonine) | No growth | 1.32 × 10⁹ |

Through this, it was confirmed that the strain of the present disclosure has various industrially useful characteristics distinguished from the *Akkermansia muciniphila* strain. The strain of the present disclosure, *"Akkermansia* HB03 *(Akkermansia* sp. HB03)", was deposited on September 11, 2018 in the Korean Culture Center of Microorganisms with the accession number KCCM12318P.

### Example 3: Preparation of tumor animal model and strain composition 3-1. Tumor animal model

6- to 8-week old male C57BL/6J JAX and BALB/c mice purchased from Daehan Biolink were used. Each of the mice was housed in a cage and was maintained under a controlled environment (12-hour light/dark cycle, light-off at 7 pm) with free access to feed and water ad libitum. Tumorigenesis was induced by injecting pancreatic cancer cells (Panc02), melanoma cells (B16F10), glioma cells (GL261), sarcoma cells (MCA205) colorectal cancer cells (MC38) and lung cancer cells (LLC) to the C57BL/6 JAX mice and injecting colorectal cancer cells (CT26) to the BALB/c mice. Specifically, 1×10⁷ cells/100 µL of Panc02, 2×10⁵ cells/100 µL of B16F10, 5×10⁵ cells/100 µL of GL261, 5×10⁵ cells/100 µL of MCA205, 5×10⁵ cells/100 µL of MC38, 5×10⁵ cells/100 µL of LLC or 5×10⁵ cells/100 µL of CT26 tumor cells were subcutaneously injected to the mice. Tumor size was measured with 2- or 3-day intervals until the end of the experiment, and tumor volume was calculated as length x width² x 0.5. In order to investigate the change in tumor size and immune environment owing to HB03 and its fragment, intestinal contents were recovered, immersed in liquid nitrogen and then kept at -80 °C. All animal experimental procedures were approved by the Animal Care and Use Committee of Korea Research Institute of Bioscience and Biotechnology.

### 3-2. Strain composition

Strain compositions HB03 (composition of *Akkermansia* sp. HB03), *Akkermansia muciniphila* (composition of *Akkermansia muciniphila* ATCC BAA-835), AKK p2261 (composition of *Akkermansia muciniphila* p2261) and *Bifidobacterium longum* (composition of *Bifidobacterium longum* ATCC BAA-999) were prepared by culturing each strain *(Akkermansia* sp. HB03, *Akkermansia muciniphila* ATCC BAA-835, *Bifidobacterium longum* ATCC BAA-999 or *Akkermansia muciniphila* p2261), which had been kept at -70 °C in 20% glycerol, at 37 °C and resuspending in a liquid culture medium at 5.0×10⁹ CFU/mL. Hereinafter, unless specified otherwise, *"Akkermansia muciniphila"* refers to the type strain *Akkermansia muciniphila* BAA-835, and "AKK p2261" refers to *Akkermansia muciniphila* p2261. HB03 and *Akkermansia muciniphila* were cultured using the modified BTTM of Example 2, AKK p2261 was cultured using a COS medium (Columbia medium + 5% sheep blood (Biomirieux^{™}); Columbia medium: polypeptone 17.0 g/L, pancreatic peptone of heart 3.0 g/L, autolytic yeast extract 3.0 g/L, corn starch 1.0 g/L, sodium chloride 5.0 g/L), and *Bifidobacterium longum* was cultured using an MRS medium (De Man, Rogosa, and Sharpe (MRS) medium).

A pasteurized strain composition (HB03 (P)) was prepared by boiling the HB03 at 70 °C for 30 minutes.

### Example 4: Administration of strain composition

100 µL of each of the strain compositions HB03, HB03 (P), *Akkermansia muciniphila,* AKK p2261 or *Bifidobacterium longum* of Example 3-2 was administered once a day by oral gavage to the animal models of colorectal cancer (CT26, MC38), lung cancer (LLC) and melanoma (B16F10) of Example 3-1 were inoculated from day 7 before tumor inoculation or from day 5 or 6 to day 18, to the animal model of glioma (GL261) from day 7 before tumor inoculation or from day 5 or 6 to day 22, to the animal model of sarcoma (MCA205) from day 7 before tumor inoculation or from day 5 or 6 to day 16, and to the animal model of pancreatic cancer (Panc02) animal model from day 7 before tumor inoculation or from day 6 to day 30.

### Example 5: Effect of administration of strain composition to tumor animal model on inhibition of tumor growth

After post-treating (administration after tumor inoculation) the tumor animal models of Example 3-1 with HB03 or HB03 (P), the difference in tumor growth was compared with a control group. The result is shown in FIG. 7.

After post-treating (administration after tumor inoculation) the tumor animal models of Example 3-1 with HB03, *Akkermansia muciniphila* or *Bifidobacterium longum,* the difference in tumor growth was compared with a control group. The result is shown in FIG. 8.

After pre-treating (administration before tumor inoculation) the tumor animal models of Example 3-1 with HB03 or HB03 (P), the difference in tumor growth was compared with a control group. The result is shown in FIG. 9.

After post-treating (administration after tumor inoculation) the tumor animal models of Example 3-1 with HB03 or AKK p2261, the difference in tumor growth was compared with a control group (PBS). The result is shown in FIG. 10.

As shown in FIG. 7 and FIG. 9, tumor growth was decreased significantly when HB03 or HB03 (P) was administered for a given period before or after the transplantation of tumor cells.

Also, as shown in FIG. 8, when anti-cancer effect was compared after administration of *Akkermansia muciniphila, Bifidobacterium longum* or HB03, the administration of HB03 increased the anti-cancer effect further. And, as shown in FIG. 10, when the anti-cancer effect was compared after administration of AKK p2261 or HB03, the administration of HB03 increased the anti-cancer effect further. Through this, it was confirmed that HB03 exhibits higher anti-cancer effect than the three other strains.

Since this result demonstrates that the intestinal microbe HB03 or HB03 (P) inhibits tumor growth as a therapeutic agent for cancer, the strain of the present disclosure HB03 can be usefully used for treating cancer.

### Example 6: Effect of co-administration of immune checkpoint inhibitor and strain composition to tumor animal model on inhibition of tumor growth

After co-administering an immune checkpoint inhibitor (aPD-1 mAb) and HB03 to the tumor animal models of Example 3-1, the difference in tumor growth was compared with a non-co-administered control group. mlgG (150 µg/mouse) and PBS (100 µL/mouse) were administered to the control group, and αPD-1 mAb (150 µg/mouse) and PBS (100 µL/mouse) or αPD-1 mAb (150 µg/mouse) and HB03 (100 µL/mouse) were administered to test groups. After tumor inoculation, 100 µL of mlgG or αPD-1 mAb was injected intravenously to the animal models of colorectal cancer (CT26), lung cancer (LLC), melanoma (B16F10) and glioma (GL261) 5 times with 2-day intervals from day 5 to day 16, and 100 µL of PBS (ctrl, control group) or HB03 was administered every day by oral gavage. The result of comparing tumor size on day 16 is shown in FIG. 11.

In order to compare the difference in tumor growth between the group to which another immune checkpoint inhibitor (αCTLA-4 mAb) and HB03 were co-administered and a non-co-administered control group, mlgG (25 µg/mouse) and PBS (100 µL/mouse) were administered to the control group and αCTLA-4 mAb (25 µg/mouse) and PBS (100 µL/mouse) or αCTLA-4 mAb (25 µg/mouse) and HB03 (100 µL/mouse) were administered to test groups. After tumor inoculation, 100 µL of mlgG or αCTLA-4 mAb was injected intravenously to the animal models of sarcoma (MCA205) and colorectal cancer (MC38) 5 times with 2-day intervals from day 5 to day 16, and 100 µL of PBS (ctrl, control group) or HB03 was administered every day by oral gavage. The result of comparing tumor size on day 16 is shown in FIG. 12.

In addition, to compare the difference in tumor growth between the group to which another immune checkpoint inhibitor (αPD-L1 mAb) and HB03 were co-administered and a non-co-administered control group, mlgG (100 µg/mouse) and PBS (100 µL/mouse) were administered to the control group and αPD-L1 mAb (100 µg/mouse) and PBS (100 µL/mouse) or αPD-L1 mAb (100 µg/mouse) and HB03 (100 µL/mouse) were administered to test groups. After tumor inoculation, 100 µL of mlgG or αPD-L1 mAb was injected intravenously to the animal model of colorectal cancer (MC38) 5 times with 2-day intervals from day 5 to day 16, and 100 µL of PBS (ctrl, control group) or HB03 was administered every day by oral gavage. The result of comparing tumor size on day 16 is shown in FIG. 13.

As shown in FIGS. 11-13, the anti-tumor effect was increased significantly when the immune checkpoint inhibitor αPD-1 mAb, αCTLA-4 mAb or αPD-L1 mAb was co-administered with HB03 as compared to when the immune checkpoint inhibitor was administered alone.

This result demonstrates that the intestinal microbe HB03 provides the advantage of inhibiting tumor growth during cancer treatment by lowering the concentration of an immune checkpoint inhibitor, thereby increasing anti-tumor effect while reducing side effects and resistance.

### Example 7: Effect of co-administration of immune checkpoint inhibitor and strain composition to resistant animal model on inhibition of tumor growth

After administering ampicillin (1µg/mL), streptomycin (5 µg/mL) and colistin (1 µg/mL) for 2 weeks to the animal models of Example 3-1 by dissolving in drinking water and then inoculating tumors, the drinking water was replaced with common drinking water. 48 hours later, after co-administering an immune checkpoint inhibitor (αPD-1 mAb) with HB03 or *Bifidobacterium longum,* the difference in tumor growth was compared with a non-co-administered group. After the tumor inoculation, mlgG or αPD-1 mAb was injected intravenously to the animal models of colorectal cancer (CT26) and melanoma (B16F10) 5 times with 2-day intervals from day 5 to day 16, and 100 µL of PBS (ctrl, control group), HB03 or *Bifidobacterium longum* was administered every day by oral gavage. The result of comparing tumor size on day 16 is shown in FIG. 14.

In addition, after administering ampicillin (1µg/mL), streptomycin (5 µg/mL) and colistin (1 µg/mL) for 2 weeks to the animal models of Example 3-1 by dissolving in drinking water and then inoculating tumors, the drinking water was replaced with common drinking water. 48 hours later, after co-administering an immune checkpoint inhibitor (αCTLA-4 mAb) with HB03, the difference in tumor growth was compared with a non-co-administered group. After the tumor inoculation, mlgG or αPD-1 mAb was injected intravenously to the animal model of colorectal cancer (MC38) 5 times with 2-day intervals from day 5 to day 16, and 100 µL of PBS (ctrl, control group) or HB03 was administered every day by oral gavage. The result of comparing tumor size on day 16 is shown in FIG. 15.

As shown in FIG. 14, when the immune checkpoint inhibitor αPD-1 mAb was co-administered with HB03 to the mice having resistance to the immune checkpoint inhibitor due to the administration of the antibiotic, anti-tumor effect was increased significantly as compared to when the immune checkpoint inhibitor was treated alone, and this effect was superior to that of the co-administration with *Akkermansia muciniphila* or *Bifidobacterium longum.*

Also, as shown in FIG. 15, when the immune checkpoint inhibitor αCTLA-4 mAb was co-administered with HB03 to the mice having resistance to the immune checkpoint inhibitor due to the administration of the antibiotic, anti-tumor effect was increased significantly as compared to when the immune checkpoint inhibitor was treated alone.

This result demonstrates that the phenomenon of creased anti-cancer effect caused by resistance to an immune checkpoint inhibitor during cancer treatment is overcome by the intestinal microbe HB03, leading to increased anti-tumor effect and inhibited tumor growth.

### Example 8: Effect of co-administration of anti-cancer agent and strain composition to tumor animal model on inhibition of tumor growth

After co-administering a chemical anti-cancer agent (5-FU: 5-fluorouracil) and a targeted anti-cancer agent (oxaliplatin) with HB03 to the tumor animal model of Example 3-1, the difference in tumor growth was compared with a non-co-administered group. Saline (100 µL/mouse) and PBS (100 µL/mouse) were administered to the control group, and 5-FU (2 mg/kg), oxaliplatin (20 mg/kg) and saline (100 µL/mouse) or 5-FU (2 mg/kg), oxaliplatin (20 mg/kg) and HB03 (100 µL/mouse) were administered to test groups. After tumor inoculation, 100 µL of 5-FU and oxaliplatin were injected intravenously to the animal model of colorectal cancer (MC38) 5 times with 2-day intervals from day 5 to day 16, and 100 µL of PBS (ctrl, control group) or HB03 was administered every day by oral gavage. The result of comparing tumor size on day 16 is shown in FIG. 16.

As shown in FIG. 16, anti-tumor effect was increased significantly when the anti-cancer agent 5-FU and oxaliplatin was co-administered with HB03 to the animal model of colorectal cancer as compared to when the anti-cancer agent was treated alone.

### Example 9: Effect of administration of strain composition to tumor cells on inhibition of tumor growth and induction of apoptosis

The genes encoding the proteins produced by the HB03 strain, HB03-01 and HB03-03, were amplified from the genomic DNA of the HB03 strain through PCR. Then, the amplified genes were cloned into a pET-30a vector (FIG. 17) and overexpressed in *E*. *coli.* After centrifuging and then resuspending the strain in a PBS buffer, the bacteria were lysed by sonication. After centrifuging again and separating the supernatant only, HB03-01 (SEQ ID NO 1) and HB03-03 (SEQ ID NO 2) proteins were purified from the supernatant using a His-taq column.

Then, it was investigated whether the purified HB03-01 and HB03-03 proteins affect the proliferation or apoptosis of cancer cells. Specifically, after treating colorectal cancer cells (CT26) and lung cancer cells (LLC) with the HB03-derived protein HB03-01 at concentrations of 0 (untreated), 0.1, 1.0 or 10 µg/mL or with HB03-03 at 10 µg/mL, the proliferation of the cancer cells and the activity of the apoptosis-regulating enzyme caspase-3 were investigated.

MTT assay was conducted to investigate the change in the proliferation of the cancer cells. The cancer cells were cultured using a DMEM medium (Gibco, USA) for 24 hours on a 96-well plate at 1×10⁴ cells/well in a 5% CO₂ incubator at 37 °C. A low-glucose condition was maintained by treating with 2-deoxyglucose (2-DG) to a final concentration of 20 mM. After culturing for 6 hours, HB03-01 was treated at a concentration of 0, 0.1, 1.0 or 10 µg/mL for 48 hours. After adding 10 µL of an MTT solution, the cells were cultured further for 2 hours. After removing the medium from the 96-well plate and dissolving a formazan formed on the plate with 100 µL of DMSO, absorbance was measured at 540 nm using an ELISA reader (Model 680, BioRad). A control group was treated with 0 µg/mL HB03-01 (untreated), and the measurement values were represented as relative cell survivability (%) with respect to the number of cells in the control group as 100%.

Western blot was conducted to investigate the activity level of caspase-3. Lung cancer cells treated with 10 µg/mL of HB03-01 or HB03-03 for 24 hours were homogenized in an ice-cold 20 mM Tris-HCI buffer (pH 7.5) containing 2 mM EDTA (ethylenediaminetetraacetic acid), 0.5 mM EGTA (ethylene glycol tetraacetic acid), 300 mM sucrose and 2 mM PMSF (phenylmethylsulfonyl fluoride). After electrophoresing 50 µg of proteins on 15% SDS-PAGE (sodium dodecyl sulfate-polyacrylamide gel electrophoresis) gel, they were transferred onto a PVDF (polyvinylidene fluoride) membrane using a semidry transfer system (Hoefer, Holliston, MA, USA). Non-specific binding was blocked by incubating with 5% non-fat dry milk. After incubating the membrane overnight at 4 °C with primary antibodies diluted to 1:500, it was incubated with secondary antibodies by binding HRP (horseradish peroxidase) diluted to 1:1000. The immune complexes were detected using an ECL detection kit (Amersham Biosciences Korea, Seoul, Korea) and auto-radiographed onto an X-ray film. The antibodies used for the western blot were actin and caspase-3. They were purchased from Santa Cruz Biotechnolgy (Santa Cruz, CA).

As shown in FIG. 18A and 18B, the HB03-derived protein HB03-01 significantly decreased the proliferation of the colorectal cancer and lung cancer cells. In addition, as shown in FIG. 18C, it activated the apoptosis-regulating enzyme (caspase-3) in the lung cancer cells together with the other HB03-derived protein HB03-03.

Through this, it was demonstrated that the proteins or metabolomes derived from the intestinal microbe HB03 create an environment favorable for cancer treatment or inhibit tumor growth.

### Example 10: Effect of administration of strain composition to tumor animal model on inhibition of tumor growth

After post-treating (administration after tumor inoculation) the tumor animal model of Example 3-1 with HB03-01 (AT1) or HB03-03 (AT3), the difference in tumor growth was compared with a control group. The result is shown in FIG. 19.

As a result, it was confirmed that HB03-01 (AT1) and HB03-03 (AT3) significantly inhibit tumor growth as compared to the control group not treated with HB03-01 (AT1) or HB03-03 (AT3).

This result demonstrates that the proteins derive from the intestinal microbe HB03 advantageously inhibit tumor growth as therapeutic agents for cancer. Accordingly, the proteins of the HB03 strain of the present disclosure can be usefully used for treatment of cancer.

The results of the above examples suggest that the presence or increased level of useful intestinal microorganisms and the level of activation or inactivation of the microorganisms affect cancer treatment and tumor growth.

In particular, the present disclosure has demonstrated that the administration of a specific microorganism, i.e., the novel beneficial microorganism of the present disclosure, facilitates the treatment of tumors and inhibits tumor growth through regulation of intestinal microbes by decreasing or suppressing other immune responses. Specifically, the novel beneficial microorganism disclosed in the present disclosure creates an environment favorable for cancer treatment and inhibits tumor growth by secreting metabolites (e.g., amino acids, nucleic acids, fatty acids, sugars, amines, glycolipids, short peptides, vitamins, hormones, etc. existing in cells, tissues or body fluids) or proteomes.

The data in the tables and graphs were expressed as mean ± SEM. P-values for investigating the statistical difference in tumor growth measurement was estimated by Student's t-test (Excel software). P < 0.05 was regarded as statistically significant: *p < 0.05, **p < 0.01, ***p < 0.001.

It will be understood by those skilled in the art to which the present disclosure belongs understand that the present disclosure can be embodied in different forms without changing the technical idea or essential features. In this regard, it should be understood that all the afore-described examples are illustrative, not limitative. It should be appreciated that all changes and modifications derived from the appended claims and their equivalents are included in the scope of the present disclosure.

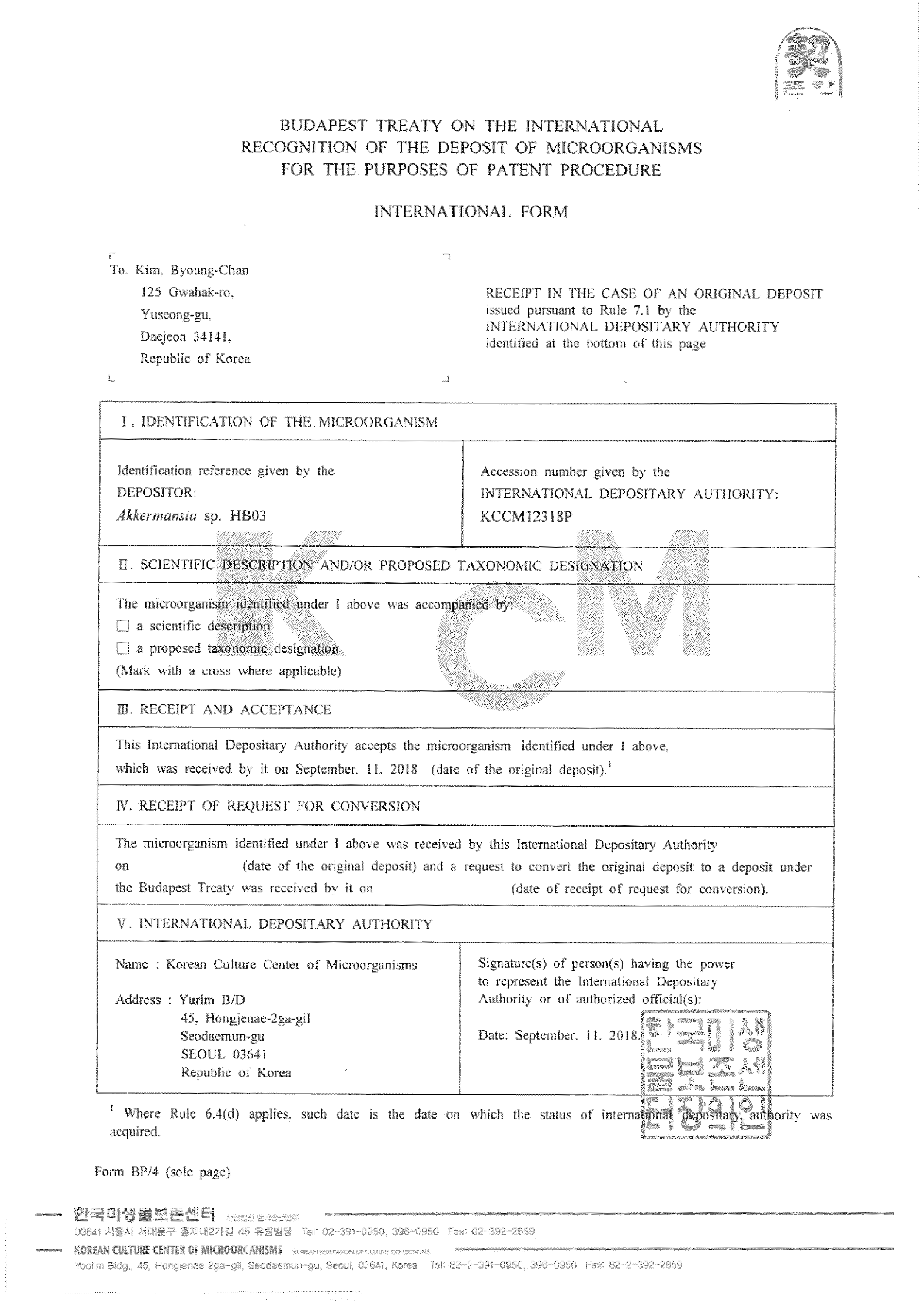

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. Kim, Byoung-Chan
125 Gwahak-ro,
Yuseong-gu,
Daejeon 34141,
Republic of Korea

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Akkermansia sp. HB03 | Accession number given by the
INTERNATIONAL DEPOSITARY AUTHORITY:
KCCM12318P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on September. 11. 2018 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: September. 11. 2018.

¹ Where Rule 6.4(d) applies, such date is the date on which the status of international depositary authority was
acquired.

Form BP/4 (sole page)

한국미생물보존센터 사단법인 한국종균협회
03641 서울시 서대문구 홍제내2가길 45 유림빌딩 Tel: 02-391-0950, 396-0950 Fax: 02-392-2859
KOREAN CULTURE CENTER OF MICROORGANISMS KOREAN FEDERATION OF CULTURE COLLECTIONS
Yoolim Bldg., 45, Hongjenae 2ga-gil, Seodaemun-gu, Seoul, 03641, Korea Tel: 82-2-391-0950, 396-0950 Fax: 82-2-392-2859

## Claims

1. A novel *Akkermansia* HB03 strain *(Akkermansia* sp. HB03) deposited with the accession number KCCM12318P.

2. A pharmaceutical composition for preventing or treating cancer, comprising one or more selected from a group consisting of the *Akkermansia* strain according to claim 1, an inactivated strain thereof, a culture thereof, a vesicle thereof, a proteome thereof and a metabolome thereof.

3. The pharmaceutical composition according to claim 2, wherein the inactivation is thermal inactivation by heating.

4. The pharmaceutical composition according to claim 2, wherein the pharmaceutical composition is administered together with an anti-cancer agent.

5. The pharmaceutical composition according to claim 4, wherein the anti-cancer agent is one or more selected from a group consisting of a chemical anti-cancer agent, a targeted anti-cancer agent and an immune checkpoint inhibitor.

6. The pharmaceutical composition according to claim 2, wherein the cancer is a cancer which exhibits resistance to an anti-cancer agent.

7. The pharmaceutical composition according to claim 6, wherein the anti-cancer agent is an immune checkpoint inhibitor.

8. The pharmaceutical composition according to claim 2, wherein the proteome is one or more protein of HB03-01 and HB03-03.

9. A method for preventing or treating cancer, comprising a step of administering the pharmaceutical composition according to any of claims 2 to 8 to an individual.

10. A food composition for preventing or alleviating cancer, comprising one or more selected from a group consisting of the *Akkermansia* strain according to claim 1, an inactivated strain thereof, a culture thereof, a vesicle thereof, a proteome thereof and a metabolome thereof.

11. The food composition according to claim 10, wherein the inactivation is thermal inactivation by heating.

12. The food composition according to claim 10, wherein the proteome is one or more protein of HB03-01 and HB03-03.

13. A use of a novel *Akkermansia* HB03 strain *(Akkermansia* sp. HB03) deposited with the accession number KCCM12318P, an inactivated strain thereof, a culture thereof, a vesicle thereof, a proteome thereof or a metabolome thereof for prevention or treatment of cancer.
